# EUROPEAN PATENT SPECIFICATION

(11) **EP 2 716 302 B1**
(45) Date of publication and mention of the grant of the patent: **27.09.2017**
(21) Application number: 12789670.2
(22) Date of filing: 25.05.2012
(51) Int. Cl.: A61K 45/00, A61K 31/137, A61K 31/138, A61K 31/15, A61K 31/335, A61K 31/343, A61K 31/451, A61K 31/4525, A61K 31/495, A61K 31/55, A61K 31/551, A61P 21/00, A61P 25/00, A61P 25/04, A61P 43/00, C07D 403/10

(54) **PROPHYLACTIC OR THERAPEUTIC AGENT FOR NEUROPATHIC PAIN ASSOCIATED WITH GUILLAIN-BARRE SYNDROME**
PROPHYLAKTISCHES ODER THERAPEUTISCHES MITTEL GEGEN NEUROPATHISCHE SCHMERZEN AUFGRUND DES GUILLAIN-BARRE-SYNDROMS
AGENT PROPHYLACTIQUE OU THÉRAPEUTIQUE POUR LA DOULEUR NEUROPATHIQUE ASSOCIÉE AU SYNDROME DE GUILLAIN-BARRÉ

(30) Priority: 25.05.2011 JP 2011116965
(43) Date of publication of application: 09.04.2014
(73) Proprietor: Kyushu University, Higashi-ku Fukuoka-shi, Fukuoka 812-8581 (JP); Nippon Chemiphar Co., Ltd., Tokyo 101-0032 (JP)
(72) Inventor: IMAI, Toshiyasu, Misato-shi Saitama 341-0005 (JP); KAWASAKI, Toru, Misato-shi Saitama 341-0005 (JP); OGAWA, Toru, Misato-shi Saitama 341-0005 (JP); INOUE, Kazuhide, Fukuoka-shi Fukuoka 812-8581 (JP)
(74) Representative: Adam, Holger
(86) International application number: PCT/JP2012/063424
(87) International publication number: WO 2012/161301

(56) References cited:
- WO-A1-2008/020651
- WO-A1-2010/093061
- WO-A1-2012/060397
- JP-A- 2006 521 308
- JP-A- 2009 057 281
- JP-A- 2009 062 278
- BROUSSEAU KRISTIN ET AL: "Pharmacologic management of anxiety and affective lability during recovery from Guillain-Barre syndrome: some preliminary observations", NEUROPSYCHIATRIC DISEASE AND TREATMENT, DOVE MEDICAL PRESS (NZ) LTD, NZ, vol. 1, no. 2, 1 January 2005 (2005-01-01) , pages 145-149, XP008091645, ISSN: 1176-6328, DOI: 10.2147/NEDT.1.2.145.61047
- ROSTASY K M ET AL: "Acute motor and sensory axonal neuropathy (AMSAN) in a 15-year-old boy presenting with severe pain and distal muscle weakness", NEUROPEDIATRICS, vol. 36, no. 4, August 2005 (2005-08), pages 260-264, XP009180379, ISSN: 0174-304X, DOI: 10.1055/S-2005-865774 [retrieved on 2005-08-24]
- OGAWA TORU ET AL: "P2X4 receptor spinal microglia is involved in mechanical allodynia in experimental autoimmune neuritis rats", JOURNAL OF PHARMACOLOGICAL SCIENCES, JAPANESE PHARMACOLOGICAL SOCIETY, TOKYO, JP, vol. 118, no. Suppl.1, 1 January 2012 (2012-01-01), page 146P, XP009180378, ISSN: 1347-8613
- NAKATA ERIKO ET AL: "Novel P2X4 antagonist NCP-308 reduces allodynia induced by scinatic nerve injury", JOURNAL OF PHARMACOLOGICAL SCIENCES, vol. 118, no. Suppl. 1, 2012, page 146P, XP009180432, & 85TH ANNUAL MEETING OF THE JAPANESE-PHARMACOLOGICAL-SOCIETY; KYOTO, JAPAN; MARCH 14 -16, 2012
- E. TOULME ET AL: "P2X4 receptors in activated C8-B4 cells of cerebellar microglial origin", THE JOURNAL OF GENERAL PHYSIOLOGY, vol. 4, no. 1, 1 April 2010 (2010-04-01), pages 4.19-353, XP055142775, ISSN: 1934-2616, DOI: 10.1006/bbrc.1996.0380
- RANDALL, D.P.: 'Treatment of Guillain-Barre Syndrome' DISEASE-A-MONTH vol. 56, no. 5, 2010, pages 279 - 287, XP027043924
- ZHANG, Z. ET AL.: 'Mechanical allodynia and spinal up-regulation of P2X4 receptor in experimental autoimmune neuritis rats' NEUROSCIENCE vol. 152, no. 2, 2008, pages 495 - 501, XP022551071

### SEQUENCE LISTING

<110> KYUSHU UNIVERSITY NIPPON CHEMIPHAR CO., LTD.
<120> PROPHYLACTIC OR THERAPEUTIC AGENT FOR NEUROPATHIC PAIN ASSOCIATED WITH GUILLAIN-BARRE SYNDROME
<130> 26533EP
<140> EP 12 789 670.2
   <141> 2012-05-25
<150> JP 2011-116965
   <151> 2011-05-25
<160> 1
<170> PatentIn version 3.5
<210> 1
   <211> 26
   <212> PRT
   <213> Bos taurus
<400> 1
   Thr Glu Ser Pro Phe Lys Asn Thr Glu Ile Ser Phe Lys Leu Gly Gln
   1               5                   10                  15
   Glu Phe Glu Glu Thr Thr Ala Asp Asn Arg
               20                  25

## Description

### Field of the invention

The present invention relates to a compound for use in preventing or treating neuropathic pain associated with Guillain-Barré syndrome.

### Background of the invention

Guillain-Barré syndrome (GBS) is a peripheral neuropathy causing an acute motor paralysis. It has been known that the crisis of GBS usually follows an inspiratory or digestive infection.

In the past, GBS has been considered to be a demyelinating polyneuropathy, which attacks peripheral nervous myelin. It has recently been recognized that an axonopathy type results in a primary axonopathy.

Further, GBS is a monophasic disease, and its typical symptom is weakened limb muscles. Sensory disorders including dysesthesia often occur, and nearly 90% of patients complain of pains such as nerve root pain, muscle pain, joint pain or the like. GBS may cause cranial neuropathies such as facial paralysis, ocular motor paralysis, and swallowing or articulation disorders. At the climax phase, GBS may cause such a respiratory muscle paralysis that the patient should use a respirator, and it may also cause a severe autonomic neuropathy including hypertension, hypotension, fluctuations in blood pressure, tachycardia, or bradycardia.

While recovery starts after an acute phase, pain may continue from the acute phase to a recovery phase. At the recovery phase, the pain is an obstacle to rehabilitation. In the past, steroids, carbamazepine, opioid, gabapentin or the like have been used for the pain at the recovery phase in a supportive care. However, the obtained analgesic effect is often insufficient.

Guillain-Barré syndrome (GBS) is a peripheral neuropathy causing an acute motor paralysis. It has been known that the crisis of GBS usually follows an infectious disease. In the past, it has been considered to be a demyelinating polyneuropathy, which attacks peripheral nervous myelin. It has been recognized that an axonopathy type results in an axonopathy.

GBS is an autoimmune disease, and its relation with each of cellular immunity and humoral immunity has been suggested in reports. It is thought that the infectious disease prior to the crisis of GBS plays an important role.

The crisis of GBS is thought to be at one to two cases per 100,000 people annually. It is observed in all the generations, and male patients are slightly more than female ones.

GBS is a monophasic disease, and its typical symptom is weakened limb muscles. Sensory disorders including dysesthesia often occur, and nearly 90% of patients complain of pains such as nerve root pain, muscle pain, joint pain or the like. At the climax phase, GBS may cause such a respiratory muscle paralysis that the patient should use a respirator, and its case may be a severe autonomic neuropathy including hypertension, hypotension, fluctuations in blood pressure, tachycardia, or bradycardia. Therefore, a systemic management is very important at an acute phase. While recovery starts after an acute phase, pain may continue from the acute phase to a recovery phase. At the recovery phase, the pain is an obstacle to rehabilitation. Steroids, carbamazepine, opioid, gabapentin or the like have been used for the pain in a supportive care. However, the obtained analgesic effect is often insufficient.

The present inventors have found that paroxetine, a diazepinedione derivative or the like having a P2X₄ receptor antagonism can be used as an agent for preventing or treating neuropathic pain, and filed patent applications (Patent documents 1 and 2).

The patent documents, however, do not clearly describe that the above-mentioned compounds are available as an agent for use in preventing or treating neuropathic pain associated with Guillain-Barré syndrome.

### Prior art documents

### Patent documents

Patent document 1: WO 2008/020651
Patent document 2: WO 2010/093061

### Summary of the invention

### Problems to be solved by the invention

It is the object of the invention to provide a compound for use in preventing or treating neuropathic pain associated with Guillain-Barré syndrome.

### Means for solving the problems

The present inventors have found that P2X₄ receptor antagonist such as paroxetine, a diazepinedione derivative or the like can be used as a compound for use in preventing or treating neuropathic pain associated with Guillain-Barré syndrome, and completed the present invention.

The present invention relates to a P2X₄ receptor antagonist as defined in the claims as an active ingredient for use in preventing or treating neuropathic pain associated with Guillain-Barré syndrome. The invention relates to a compound having the following formula (I) or a pharmacologically acceptable salt thereof for use in preventing or treating neuropathic pain associated with Guillain-Barré syndrome: 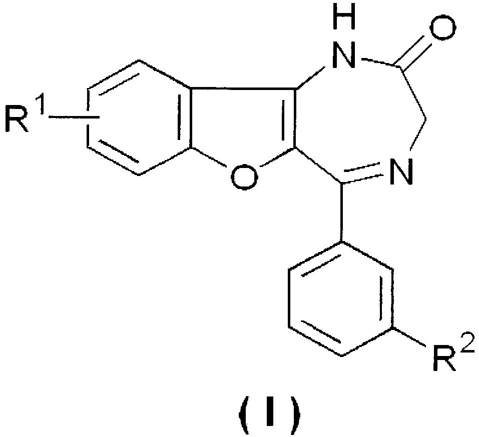
wherein R¹ is a halogen atom; and
R² is hydrogen, a halogen atom, nitro, cyano, -C(O)-OR³, -C(O)-NR⁴R⁵, -SO₂-OR³, or -SO₂-NR⁴R⁵, wherein each of R³ , R⁴, and R⁵ is hydrogen or a C₁₋₆ alkyl group; or in the alternative
R¹ is hydrogen; and
R² is a halogen atom, nitro, cyano, -C(O)-OR³, - C(O)-NR⁴R⁵, -SO₂-OR³, or -SO₂-NR⁴R⁵, wherein each of R³, R⁴, and R⁵ is hydrogen or a C₁₋₆ alkyl group.

The invention further relates to a compound having the following formula (Ia) or a pharmacologically acceptable salt thereof for use in preventing or treating neuropathic pain associated with Guillain-Barré syndrome: 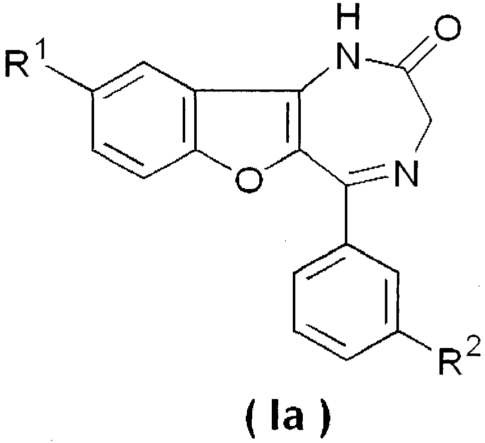
wherein R¹ is chloro or bromo; and
R² is hydrogen, chloro, bromo, nitro, or cyano; or in the alternative
R¹ is hydrogen; and
R² is chloro, bromo, nitro, or cyano.

The invention further relates to a compound having the following formula (III) or a pharmacologically acceptable salt thereof for use in preventing or treating neuropathic pain associated with Guillain-Barré syndrome: 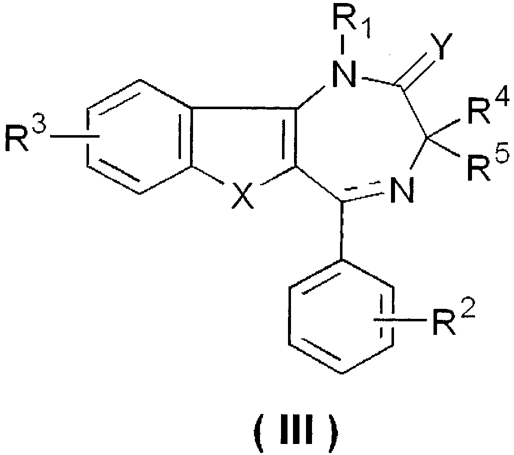
wherein X is S or CH₂;
Y is O, S, or NH;
R¹ is hydrogen, a C₁₋₈ alkyl group, a C₁₋₈ alkyl group having one or more halogen atoms, an aralkyl group comprising a C₁₋₆ alkyl moiety and a C₆₋₁₀ aryl moiety, a C₂₋₈ alkenyl group, carboxymethyl, or an alkoxycarbonylmethyl group comprising a C₁₋₈ alkoxy moiety;
each of R² and R³ independently is hydrogen, a C₁₋₈ alkyl group, a C₁₋₈ alkoxy group, a C₁₋₈ alkyl group having one or more halogen atoms, a C₁₋₈ alkoxy group having one or more halogen atoms, a halogen atom, amino, carboxyl, hydroxyl, nitro, cyano, a C₂₋₈ acyl group, a C₆₋₁₀ aryl group, or a five-membered or six-membered heterocyclic group;
each of R⁴ and R⁵ independently is hydrogen, a C₁₋₈ alkyl group, or a C₁₋₈ alkyl group having one or more halogen atoms; and
the double line consisting of a broken line and a solid line is a single bond or a double bond.

The invention further relates to a compound having the following formula (IV) or a pharmacologically acceptable salt thereof for use in preventing or treating neuropathic pain associated with Guillain-Barré syndrome: 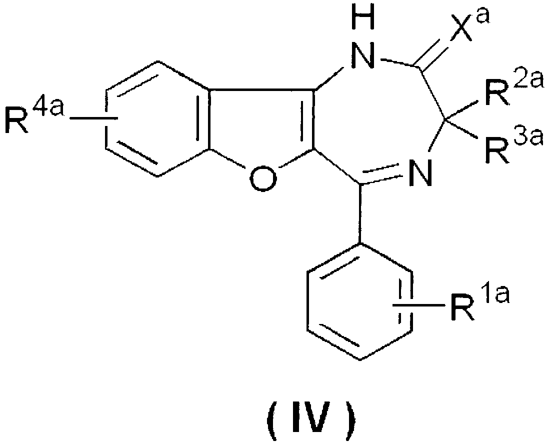
wherein X^{a} is O, S, or NH;
R^{1a} is hydroxyl, tetrazolyl, N(R^{5a})(R^{6a}), a C₂₋₈ alkenyl group, a C₂₋₈ alkynyl group, a C₁₋₈ alkyl group having one or more halogen atoms, a C₁₋₈ alkoxy group having one or more halogen atoms, or a C₆₋₁₀ aryl group, wherein R^{5a} is hydrogen or a C₁₋₈ alkyl group, and R^{6a} is hydrogen, a C₁₋₈ alkyl group, or a C₂₋₈ acyl group;
each of R^{2a} and R^{3a} independently is hydrogen, a C₁₋₈ alkyl group, or a C₁₋₈ alkyl group having one or more halogen atoms; and
R^{4a} is hydrogen, a C₁₋₈ alkyl group, a C₁₋₈ alkoxy group, a C₁₋₈ alkyl group having one or more halogen atoms, a halogen atom, hydroxyl, nitro, amino, carboxyl, tetrazolyl, cyano, a C₆₋₁₀ aryl group, or a five-membered or six-membered heterocyclic group.

The invention further relates to a compound having the following formula (V) or a pharmacologically acceptable salt thereof for use in preventing or treating neuropathic pain associated with Guillain-Barré syndrome: 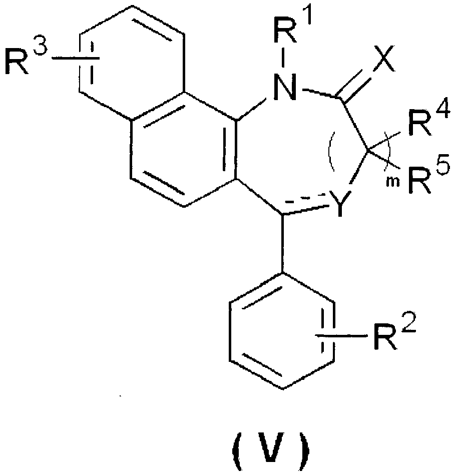
wherein X is 0, S, or NH;
Y is N or NR⁶, wherein R⁶ is hydrogen or a C₁₋₈ alkyl group;
R¹ is hydrogen, a C₁₋₈ alkyl group, a C₂₋₈ alkenyl group, a C₁₋₈ alkyl group having one to three halogen atoms, or an alkyl group having phenyl;
R² is a C₁₋₈ alkyl group, a C₁₋₈ alkoxy group, a C₁₋₈ alkyl group having one to three halogen atoms, hydroxyl, nitro, amino, carboxyl, tetrazolyl, or cyano;
R³ is hydrogen, a C₁₋₈ alkyl group, a C₁₋₈ alkoxy group, a C₁₋₈ alkyl group having one to three halogen atoms, a halogen atom, hydroxyl, nitro, amino, carboxyl, tetrazolyl, or cyano;
each of R⁴ and R⁵ independently is hydrogen, a C₁₋₈ alkyl group, or a C₁₋₈ alkyl group having one to three halogen atoms;
m is 1 or 2;
when Y is N, the double line consisting of a solid line and a broken line is a double bond; and
when Y is NR⁶, the double line consisting of a solid line and a broken line is a single bond.

The invention further relates to a compound having the following formula (Va) or a pharmacologically acceptable salt thereof for use in preventing or treating neuropathic pain associated with Guillain-Barré syndrome: 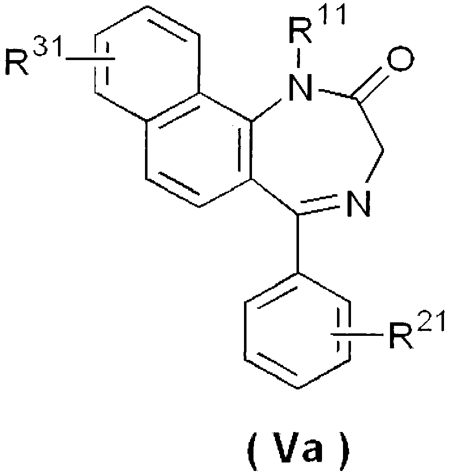
wherein R¹¹ is hydrogen or a C₁₋₈ alkyl group;
R²¹ is a C₁₋₈ alkyl group, a C₁₋₈ alkoxy group, a C₁₋₈ alkyl group having one to three halogen atoms, or hydroxyl; and
R³¹ is hydrogen or a halogen atom.

The invention further relates to a compound having the following formula (VIII) or a pharmacologically acceptable salt thereof for use in preventing or treating neuropathic pain associated with Guillain-Barré syndrome: 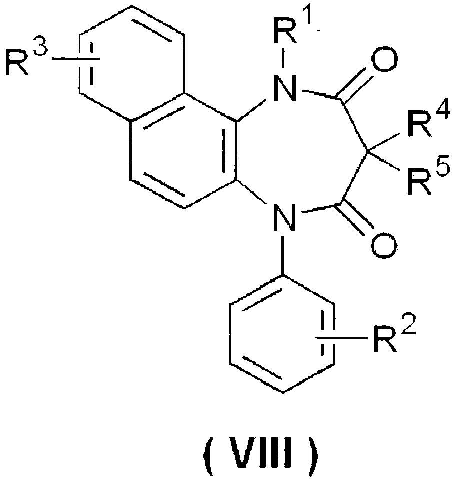
wherein R¹ is hydrogen, a C₁₋₈ alkyl group, a C₂₋₈ alkenyl group, a C₁₋₈ alkyl group having one to three halogen atoms, or a C₁₋₃ alkyl group having phenyl;
R² is hydrogen, a C₁₋₈ alkyl group, a C₁₋₈ alkoxy group, a C₁₋₈ alkyl group having one to three halogen atoms, a C₁₋₈ alkoxy group having one to three halogen atoms, a halogen atom, hydroxyl, nitro, cyano, amino, a C₁₋₈ alkylamino group, a C₂₋₈ dialkylamino group, a C₂₋₈ acylamino group, a C₂₋₈ acylamino group having one to three halogen atoms, a C₁₋₈ alkylsulfonylamino group, carboxyl, a C₂₋₈ acyl group, an alkoxycarbonyl group comprising a C₁₋₈ alkoxy moiety, carbamoyl, a C₁₋₈ alkylthio group, a C₁₋₈ alkylsulfinyl group, a C₁₋₈ alkylsulfonyl group, or sulfamoyl;
R³ is hydrogen, a C₁₋₈ alkyl group, a C₁₋₈ alkoxy group, a C₁₋₈ alkyl group having one to three halogen atoms, a C₁₋₈ alkoxy group having one to three halogen atoms, a halogen atom, hydroxyl, nitro, cyano, amino, carboxyl, a C₂₋₈ acyl group, or an alkoxycarbonyl group comprising a C₁₋₈ alkoxy moiety; and
each of R⁴ and R⁵ independently is hydrogen, a C₁₋₈ alkyl group, or a C₁₋₈ alkyl group having one to three halogen atoms.

The invention further relates to 5-[3-(1H-tetrazol-5-yl)phenyl]-1H-naphtho[1,2-b][1,4]diazepine-2,4(3H,5H)-dione potassium salt for use in preventing or treating neuropathic pain associated with Guillain-Barré syndrome.

### Brief descriptions of the drawings

Fig. 1 shows immunostaining images of Iba1-positive cell signals (upper figures) or P2X₄ receptor-positive signals (lower figures). The left figures show controls, and the right figures show EAN models.
Fig. 2 shows influence of preventive administration of the compound A on pain threshold of EAN rat model.
Fig. 3 shows influence of therapeutic administration of the compound A on pain threshold of EAN rat model.

### The embodiments of the invention

The present invention is described below in more detail.

The compounds for use in preventing or treating neuropathic pain associated with Guillain-Barré syndrome according to the invention include the following compounds.
(2) A compound having the following formula (I) or a pharmacologically acceptable salt thereof: 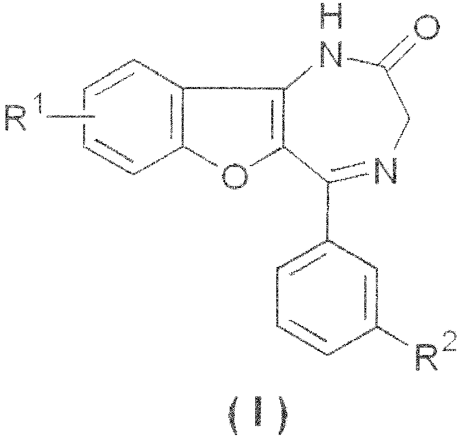
   wherein R¹ is a halogen atom; and
   R² is hydrogen, a halogen atom, nitro, cyano, -C(O)-OR³, -C(O)-NR⁴R⁵, -SO₂-OR³, or -SO₂-NR⁴R⁵, wherein each of R³, R⁴, and R⁵ is hydrogen or a C₁₋₆ alkyl group; or in the alternative
   R¹ is hydrogen; and
   R² is a halogen atom, nitro, cyano, -C(O)-OR³, C(O)-NR⁴R⁵, -SO₂-OR³, or -SO₂-NR⁴R⁵, wherein each of R³, R⁴, and R⁵ is hydrogen or a C₁₋₆ alkyl group.
(3) A compound having the formula (I) described in (2) or a pharmacologically acceptable salt thereof:
   wherein R¹ is chloro or bromo; and
   R² is hydrogen, chloro, bromo, nitro, cyano, -C(O)-OR³, or -C(O)-NR⁴R⁵, wherein each of R³, R⁴, and R⁵ is hydrogen or a C₁₋₄ alkyl group; or in the alternative
   R¹ is hydrogen; and
   R² is chloro, bromo, nitro, cyano, -C(O)-OR³, or - C(O)-NR⁴R⁵, wherein each of R³, R⁴, and R⁵ is hydrogen or a C₁₋₄ alkyl group.
(4) A compound having the following formula (Ia) or a pharmacologically acceptable salt thereof: 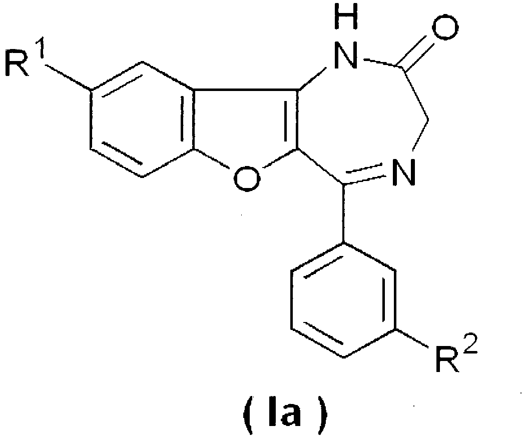
   wherein R¹ is chloro or bromo; and
   R² is hydrogen, chloro, bromo, nitro, or cyano; or in the alternative
   R¹ is hydrogen; and
   R² is chloro, bromo, nitro, or cyano.
(8) A compound having the following formula (III) or a pharmacologically acceptable salt thereof: 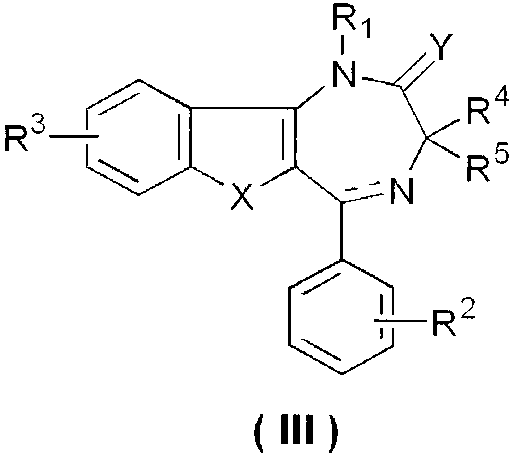
   wherein X is S or CH₂;
   Y is 0, S, or NH;
   R¹ is hydrogen, a C₁₋₈ alkyl group, a C₁₋₈ alkyl group having one or more halogen atoms, an aralkyl group comprising a C₁₋₆ alkyl moiety and a C₆₋₁₀ aryl moiety, a C₂₋₈ alkenyl group, carboxymethyl, or an alkoxycarbonylmethyl group comprising a C₁₋₈ alkoxy moiety;
   each of R² and R³ independently is hydrogen, a C₁₋₈ alkyl group, a C₁₋₈ alkoxy group, a C₁₋₈ alkyl group having one or more halogen atoms, a C₁₋₈ alkoxy group having one or more halogen atoms, a halogen atom, amino, carboxyl, hydroxyl, nitro, cyano, a C₂₋₈ acyl group, a C₆₋₁₀ aryl group, or a five-membered or six-membered heterocyclic group;
   each of R⁴ and R⁵ independently is hydrogen, a C₁₋₈ alkyl group, or a C₁₋₈ alkyl group having one or more halogen atoms; and
   the double line consisting of a broken line and a solid line is a single bond or a double bond.
(9) A compound having the formula (III) described in (8) or a pharmacologically acceptable salt thereof, wherein X is S.
(10) A compound having the formula (III) described in (8) or a pharmacologically acceptable salt thereof, wherein Y is O.
(11) A compound having the formula (III) described in (8) or a pharmacologically acceptable salt thereof, wherein R¹ is hydrogen or a C₁₋₈ alkyl group.
(12) A compound having the formula (III) described in (8) or a pharmacologically acceptable salt thereof, wherein each of R² and R³ independently is hydrogen, a C₁₋₈ alkyl group, a C₁₋₉ alkoxy group, a C₁₋₈ alkyl group having one or more halogen atoms, a C₁₋₈ alkoxy group having one or more halogen atoms, a halogen atom, amino, carboxyl, hydroxyl, nitro, or cyano.
(13) A compound having the formula (III) described in (8) or a pharmacologically acceptable salt thereof, wherein R³ is hydrogen, and R² is a halogen atom or hydroxyl.
(14) A compound having the formula (III) described in (8) or a pharmacologically acceptable salt thereof, wherein R² substitutes at meta-position.
(15) A compound having the formula (III) described in (8) or a pharmacologically acceptable salt thereof, wherein each of R⁴ and R⁵ is hydrogen.
(16) A compound having the formula (III) described in (8) or a pharmacologically acceptable salt thereof, wherein the double line consisting of a broken line and a solid line is a double bond.
(17) A compound having the following formula (IV) or a pharmacologically acceptable salt thereof: 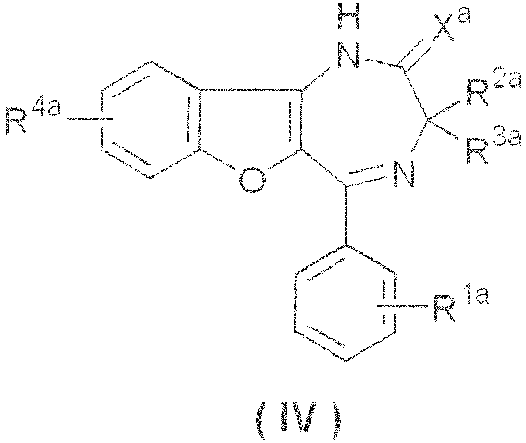
   wherein X^{a} is O, S, or NH;
   R^{1a} is hydroxyl, tetrazolyl, N(R^{5a}) (R^{6a}), a C₂₋₈ alkenyl group, a C₂₋₈ alkynyl group, a C₁₋₈ alkyl group having one or more halogen atoms, a C₁₋₈ alkoxy group having one or more halogen atoms, or a C₆₋₁₀ aryl group, wherein R^{5a} is hydrogen or a C₁₋₈ alkyl group, and R^{6a} is hydrogen, a C₁₋₈ alkyl group, or a C₂₋₈ acyl group;
   each of R^{2a} and R^{3a} independently is hydrogen, a C₁₋₈ alkyl group, or a C₁₋₈ alkyl group having one or more halogen atoms; and
   R^{4a} is hydrogen, a C₁₋₈ alkyl group, a C₁₋₈ alkoxy group, a C₁₋₈ alkyl group having one or more halogen atoms, a halogen atom, hydroxyl, nitro, amino, carboxyl, tetrazolyl, cyano, a C₆₋₁₀ aryl group, or a five-membered or six-membered heterocyclic group.
(18) A compound having the formula (IV) described in (17) or a pharmacologically acceptable salt thereof, wherein X^{a} is O.
(19) A compound having the formula (IV) described in (17) or a pharmacologically acceptable salt thereof, wherein R^{1a} is hydroxyl, amino, a C₁₋₈ alkylamino group, a C₂₋₁₂ dialkylamino group, a C₁₋₈ alkyl group having one or more halogen atoms, or phenyl.
(20) A compound having the formula (IV) described in (17) or a pharmacologically acceptable salt thereof, wherein R^{1a} substitutes at meta-position.
(21) A compound having the formula (IV) described in (17) or a pharmacologically acceptable salt thereof, wherein each of R^{2a} and R^{3a} is hydrogen.
(22) A compound having the formula (IV) described in (17) or a pharmacologically acceptable salt thereof, wherein R^{4a} is hydrogen.
(37) A compound having the following formula (V) or a pharmacologically acceptable salt thereof: 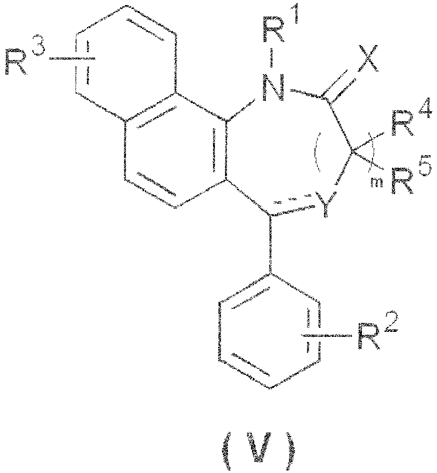
   wherein X is O, S, or NH;
   Y is N or NR⁶, wherein R⁶ is hydrogen or a C₁₋₈ alkyl group;
   R¹ is hydrogen, a C₁₋₈ alkyl group, a C₂₋₈ alkenyl group, a C₁₋₈ alkyl group having one to three halogen atoms, or an alkyl group having phenyl;
   R² is a C₁₋₈ alkyl group, a C₁₋₈ alkoxy group, a C₁₋₈ alkyl group having one to three halogen atoms, hydroxyl, nitro, amino, carboxyl, tetrazolyl, or cyano;
   R³ is hydrogen, a C₁₋₈ alkyl group, a C₁₋₈ alkoxy group, a C₁₋₈ alkyl group having one to three halogen atoms, a halogen atom, hydroxyl, nitro, amino, carboxyl, tetrazolyl, or cyano;
   each of R⁴ and R⁵ independently is hydrogen, a C₁₋₈ alkyl group, or a C₁₋₈ alkyl group having one to three halogen atoms;
   m is 1 or 2;
   when Y is N, the double line consisting of a solid line and a broken line is a double bond; and
   when Y is NR⁶, the double line consisting of a solid line and a broken line is a single bond.
(38) A compound having the formula (V) described in (37) or a pharmacologically acceptable salt thereof, wherein m is 1.
(39) A compound having the formula (V) described in (37) or a pharmacologically acceptable salt thereof, wherein X is O.
(40) A compound having the formula (V) described in (37) or a pharmacologically acceptable salt thereof, wherein Y is N.
(41) A compound having the formula (V) described in (37) or a pharmacologically acceptable salt thereof, wherein R¹ is hydrogen or a C₁₋₈ alkyl group.
(42) A compound having the formula (V) described in (37) or a pharmacologically acceptable salt thereof, wherein R¹ is hydrogen.
(43) A compound having the formula (V) described in (37) or a pharmacologically acceptable salt thereof, wherein each of R⁴ and R⁵ is hydrogen.
(44) A compound having the formula (V) described in (37) or a pharmacologically acceptable salt thereof, wherein R² is a C₁₋₈ alkyl group, a C₁₋₈ alkoxy group, a C₁₋₈ alkyl group having one to three halogen atoms, or hydroxyl.
(45) A compound having the formula (V) described in (37) or a pharmacologically acceptable salt thereof, wherein R² is a C₁₋₈ alkoxy group or hydroxyl.
(46) A compound having the formula (V) described in (37) or a pharmacologically acceptable salt thereof, wherein R³ is hydrogen or a halogen atom.
(47) A compound having the formula (V) described in (37) or a pharmacologically acceptable salt thereof, wherein R³ is hydrogen.
(48) A compound having the following formula (Va) or a pharmacologically acceptable salt thereof: 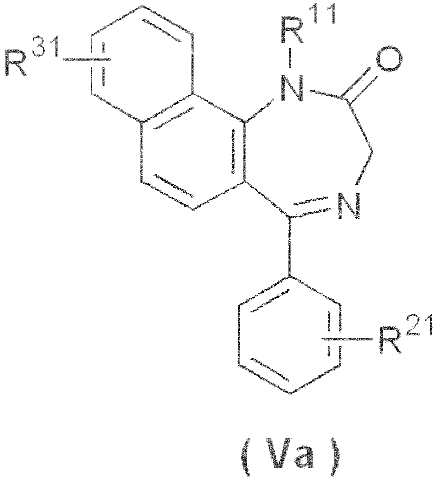
   wherein R¹¹ is hydrogen or a C₁₋₈ alkyl group;
   R²¹ is a C₁₋₈ alkyl group, a C₁₋₈ alkoxy group, a C₁₋₈ alkyl group having one to three halogen atoms, or hydroxyl; and
   R³¹ is hydrogen or a halogen atom.
(49) A compound having the formula (Va) described in (48) or a pharmacologically acceptable salt thereof, wherein R¹¹ is hydrogen.
(50) A compound having the formula (Va) described in (48) or a pharmacologically acceptable salt thereof, wherein R²¹ is a C₁₋₈ alkoxy group or hydroxyl.
(51) A compound having the formula (Va) described in (48) or a pharmacologically acceptable salt thereof, wherein R³¹ is hydrogen.
(52) 5-(3-methoxyphenyl)-1,3-dihydro-2H-naphtho[1,2-e]-1,4-diazepin-2-one,
   5-(3-hydroxyphenyl)-1,3-dihydro-2H-naphtho[1,2-e]-1,4-diazepin-2-one,
   5-(4-methoxyphenyl)-1,3-dihydro-2H-naphtho[1,2-e]-1,4-diazepin-2-one,
   5-(4-hydroxyphenyl)-1,3-dihydro-2H-naphtho[1,2-e]-1,4-diazepin-2-one,
   5-(4-methylphenyl)-1,3-dihydro-2H-naphtho[1,2-e]-1,4-diazepin-2-one,
   5-(2-methoxyphenyl)-1,3-dihydro-2H-naphtho[1,2-e]-1,4-diazepin-2-one,
   5-(2-hydroxyphenyl)-1,3-dihydro-2H-naphtho[1,2-e]-1,4-diazepin-2-one,
   5-(3,4-dimethoxyphenyl)-1,3-dihydro-2H-naphtho[1,2-e]-1,4-diazepin-2-one,
   5-(3,4-dihydroxyphenyl)-1,3-dihydro-2H-naphtho[1,2-e]-1,4-diazepin-2-one, or
   a pharmacologically acceptable salt thereof.
(95) A compound having the following formula (VIII) or a pharmacologically acceptable salt thereof: 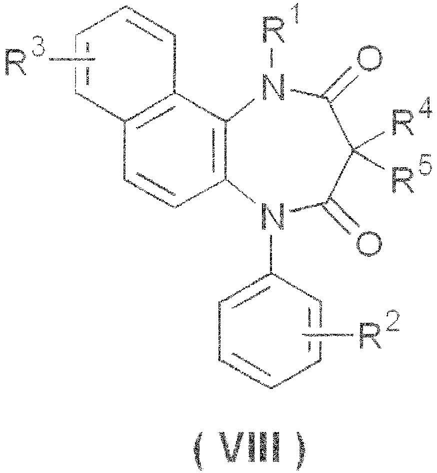
   wherein R¹ is hydrogen, a C₁₋₈ alkyl group, a C₂₋₈ alkenyl group, a C₁₋₈ alkyl group having one to three halogen atoms, or a C₁₋₃ alkyl group having phenyl;
   R² is hydrogen, a C₁₋₈ alkyl group, a C₁₋₈ alkoxy group, a C₁₋₈ alkyl group having one to three halogen atoms, a C₁₋₈ alkoxy group having one to three halogen atoms, a halogen atom, hydroxyl, nitro, cyano, amino, a C₁₋₈ alkylamino group, a C₂₋₈ dialkylamino group, a C₂₋₈ acylamino group, a C₂₋₈ acylamino group having one to three halogen atoms, a C₁₋₈ alkylsulfonylamino group, carboxyl, a C₂₋₈ acyl group, an alkoxycarbonyl group comprising a C₁₋₈ alkoxy moiety, carbamoyl, a C₁₋₈ alkylthio group, a C₁₋₈ alkylsulfinyl group, a C₁₋₈ alkylsulfonyl group, or sulfamoyl;
   R³ is hydrogen, a C₁₋₈ alkyl group, a C₁₋₈ alkoxy group, a C₁₋₈ alkyl group having one to three halogen atoms, a C₁₋₈ alkoxy group having one to three halogen atoms, a halogen atom, hydroxyl, nitro, cyano, amino, carboxyl, a C₂₋₈ acyl group, or an alkoxycarbonyl group comprising a C₁₋₈ alkoxy moiety; and
   each of R⁴ and R⁵ independently is hydrogen, a C₁₋₈ alkyl group, or a C₁₋₈ alkyl group having one to three halogen atoms.
(96) A compound having the formula (VIII) described in (95) or a pharmacologically acceptable salt thereof, wherein R¹ is hydrogen or a C₁₋₈ alkyl group.
(97) A compound having the formula (VIII) described in (95) or a pharmacologically acceptable salt thereof, wherein R¹ is hydrogen.
(98) A compound having the formula (VIII) described in (95) or a pharmacologically acceptable salt thereof, wherein R⁴ is hydrogen, and R⁵ is hydrogen or a C₁₋₈ alkyl group.
(99) A compound having the formula (VIII) described in (95) or a pharmacologically acceptable salt thereof, wherein each of R⁴ and R⁵ is hydrogen.
(100) A compound having the formula (VIII) described in (95) or a pharmacologically acceptable salt thereof, wherein R² is a C₁₋₈ alkoxy group, hydroxyl, carboxyl, cyano, or an alkoxycarbonyl group comprising a C₁₋₈ alkoxy moiety.
(101) A compound having the formula (VIII) described in (95) or a pharmacologically acceptable salt thereof, wherein R² is a C₁₋₈ alkoxy group or hydroxyl.
(102) A compound having the formula (VIII) described in (95) or a pharmacologically acceptable salt thereof, wherein R³ is hydrogen.
(103) A compound having the following formula (IX) or a pharmacologically acceptable salt thereof: 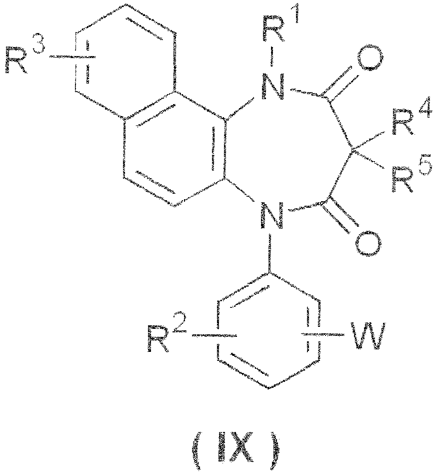
   wherein R¹ is hydrogen, a C₁₋₈ alkyl group, a C₂₋₈ alkenyl group, a C₁₋₈ alkyl group having one to three halogen atoms, or a C₁₋₃ alkyl group having phenyl;
   each of R² and R³ independently is hydrogen, a C₁₋₈ alkyl group, a C₁₋₈ alkoxy group, a C₁₋₈ alkyl group having one to three halogen atoms, a C₁₋₈ alkoxy group having one to three halogen atoms, a halogen atom, hydroxyl, nitro, cyano, amino, a C₁₋₈ alkylamino group, a C₂₋₈ dialkylamino group, a C₂₋₈ acylamino group, a C₂₋₈ acylamino group having one to three halogen atoms, a C₁₋₈ alkylsulfonylamino group, carboxyl, a C₂₋₈ acyl group, an alkoxycarbonyl group comprising a C₁₋₈ alkoxy moiety, carbamoyl, a C₁₋₈ alkylthio group, a C₁₋₈ alkylsulfinyl group, a C₁₋₈ alkylsulfonyl group, or sulfamoyl;
   each of R⁴ and R⁵ independently is hydrogen, a C₁₋₈ alkyl group, a C₁₋₈ alkyl group having one to three halogen atoms, or a C₁₋₃ alkyl group having phenyl; and
   W is a five-membered or six-membered heterocyclic ring optionally having one or more substituents and comprising one to four nitrogen atoms as the members of the ring.
(104) A compound having the formula (IX) described in (103) or a pharmacologically acceptable salt thereof, wherein W is tetrazole, 1,2,4-triazole, 1,2,3-triazole, 1,2,4-oxadiazole, pyrazole, or imidazole, each of which optionally has one or more substituents selected from the group consisting of a C₁₋₈ alkyl group, a C₁₋₈ alkyl group having one to three halogen atoms, a halogen atom, cyano, oxo, and thioxo.
(105) A compound having the formula (IX) described in (103) or a pharmacologically acceptable salt thereof, wherein W is tetrazole, 1,2,4-triazole, or 1,2,3-triazole, each of which optionally has one or more substituents selected from the group consisting of a C₁₋₈ alkyl group, a C₁₋₈ alkyl group having one to three halogen atoms, a halogen atom, and cyano.
(106) A compound having the formula (IX) described in (103) or a pharmacologically acceptable salt thereof, wherein W is 5-oxo-1,2,4-oxadiazole or 5-thioxo-1,2,4-oxadiazole.
(107) A compound having the formula (IX) described in (103) or a pharmacologically acceptable salt thereof, wherein W is tetrazole.
(108) A compound having the formula (IX) described in (103) or a pharmacologically acceptable salt thereof, wherein R¹ is hydrogen or a C₁₋₈ alkyl group.
(109) A compound having the formula (IX) described in (103) or a pharmacologically acceptable salt thereof, wherein R¹ is hydrogen.
(110) A compound having the formula (IX) described in (103) or a pharmacologically acceptable salt thereof, wherein R⁴ is hydrogen, and R⁵ is hydrogen or a C₁₋₈ alkyl group.
(111) A compound having the formula (IX) described in (103) or a pharmacologically acceptable salt thereof, wherein each of R⁴ and R⁵ is hydrogen.
(112) A compound having the formula (IX) described in (103) or a pharmacologically acceptable salt thereof, wherein R² is hydrogen, a C₁₋₈ alkyl group, a C₁₋₈ alkoxy group, a C₁₋₈ alkyl group having one to three halogen atoms, a C₁₋₈ alkoxy group having one to three halogen atoms, a halogen atom, hydroxyl, nitro, cyano, amino, carboxyl, a C₂₋₈ acyl group, or an alkoxycarbonyl group comprising a C₁₋₈ alkoxy moiety.
(113) A compound having the formula (IX) described in (103) or a pharmacologically acceptable salt thereof, wherein R² is hydrogen.
(114) A compound having the formula (IX) described in (103) or a pharmacologically acceptable salt thereof, wherein R³ is hydrogen, a C₁₋₈ alkyl group, a C₁₋₈ alkoxy group, a C₁₋₈ alkyl group having one to three halogen atoms, a C₁₋₈ alkoxy group having one to three halogen atoms, a halogen atom, hydroxyl, nitro, cyano, amino, carboxyl, a C₂₋₈ acyl group, or an alkoxycarbonyl group comprising a C₁₋₈ alkoxy moiety.
(115) A compound having the formula (IX) described in (103) or a pharmacologically acceptable salt thereof, wherein R³ is hydrogen.
(116) 5-[3-(1H-tetrazol-5-yl)phenyl]-1H-naphtho[1,2-b][1,4]diazepine-2,4(3H,5H)-dione potassium salt.

The above-mentioned compounds can be prepared according to known processes. For example, the compounds described in (2) to (4) can be prepared according to a process described in WO 2004/085440. The compounds described in (8) to (16) can be prepared according to a process described in WO 2007/072974. The compounds described in (17) to (22) can be prepared according to a process described in WO 2007/074970. The compounds described in (37) to (52) can be prepared according to a process described in WO 2008/023847. The compounds described in (95) to (102) can be prepared according to a process described in WO 2010/090300. The compounds described in (103) to (116) can be prepared according to a process described in WO 2010/093061.

The above-mentioned WO 2004/085440, WO 2007/072974, WO 2007/074970, WO 2008/023847, WO 2010/090300, WO 2010/093061, WO 2007/049825, and WO 2008/020651 describe that the compounds described in (2) to (116) have P2X₄ receptor antagonism.

The pharmacologically acceptable salts in the active ingredients used in the present invention include a salt with an acid (e.g., hydrochloric acid, acetic acid, benzoic acid, fumaric acid, besylic acid), an alkali metal (e.g., sodium, potassium, lithium), or an amine.

The active ingredients used in the present invention can be a geometrical (cis-trans) isomer or an optical isomer such as an optically active substance and racemic modification, each of which is included within the scope of the invention.

Hydrates can also be used as the active ingredients of the present invention.

The results of the pharmacological experiments are described below.

The effect of P2X₄ receptor antagonist on neuropathic pain was examined using an experimental autoimmune neuritis (EAN) rat model (Examples 3 and 4), which has been used as an experimental model for Guillain-Barré syndrome (GBS).

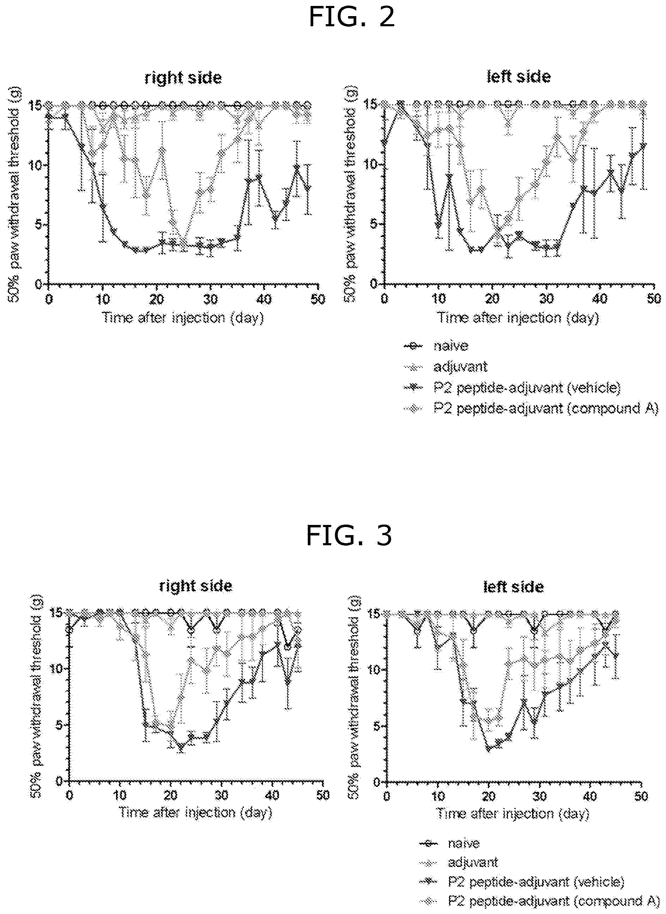

The results of Examples 3 and 4 as well as Figs. 2 and 3 show analgesic activities of the compound A, which has P2X₄ receptor antagonism, on neuropathic pain originated from EAN. The results suggest that P2X₄ receptor plays a major role in neuropathic pain associated with Guillain-Barré syndrome.

Further, it is suggested using the EAN rat model in the acute phase of autoimmune neuritis that spinal microglial cells proliferate and proliferation and activation of expression of P2X₄ receptor play important roles in causing the GBS neuropathic pain. Therefore, it is furthermore indicated that P2X₄ receptor antagonist can be an effective therapeutic agent for the GBS neuropathic pain.

The preventive or therapeutic agent used in the present invention can be administered to human beings by ordinary administration methods such as oral administration or parenteral administration.

The compound can be granulated in ordinary manners for the preparation of pharmaceuticals. For instance, the compound can be processed to give tablets, granule, powder, capsule, suspension, injection, suppository, and the like.

Ordinary additives such as vehicles, disintegrators, binders, lubricants, and dyes are used for the preparation of these pharmaceuticals such as tablets. As the vehicles, lactose, D-mannitol, crystalline cellulose, and glucose can be mentioned. Further, there can be mentioned starch and carboxymethylcellulose calcium (CMC-Ca) as the disintegrators, magnesium stearate and talc as the lubricants, and hydroxylpropylcellulose (HPC), gelatin and polyvinylpyrrolidone (PVP) as the binders. The preparation of an injection can be made using solvents, stabilizers, dissolution-aids, suspensions, emulsifiers, soothing agents, buffers, or preservatives.

The compound used in the invention can be administered to an adult generally in an amount of approximately 0.01 mg to 100 mg a day by parenteral administration and 1 mg to 2,000 mg a day by oral administration. The dosage can be adjusted in consideration of age and conditions of the patient.

### Examples

### [Example 1]

### (Experimental procedure)

P2X₄ receptor antagonisms of the compound A (5-[3-(1H-tetrazol-5-yl)phenyl]-1H-naphtho[1,2-b][1,4]diaze-pine-2,4(3H,5H)-dione potassium salt and paroxetine (reference compound) were measured as described below.

ATP receptors (human P2X₄) were introduced into 1321N1 cells, and used as a stable ATP receptor-expressing system. The obtained P2X₉ expressing 1321N1 cells were plated in a 96-well assay plate, and cultured 24 hours at 37°C in an atmosphere of 5% CO₂ for calcium assay. Fura-2 AM calcium fluorescent indicator was dissolved in an extracellular solution for calcium imaging. The obtained solution was loaded onto the plated cells, and placed at room temperature for 45 minutes to introduce Fura-2 AM into the cells. The fluorescence was detected by EnVision micro plate reader (PerkinElmer). The cells were alternatively illuminated with two excitations wavelengths (lights through 340 nm and 380 nm filters) via xenon lamp, and the emitted fluorescence was measured at 510 nm. The fluorescence changes were monitored to determine the fluorescence ratio (F340/F380) as the index of intracellular calcium change. Measurements were conducted by adding 1 µM ATP to each well, and monitoring the ATP induced intracellular calcium responses with the passage of time. Tested compounds were treated to cells 15 min before the addition of ATP, and the inhibitory activities of compounds were calculated by comparing the calcium response with control in the absence of tested compound.

### (Experimental results)

**TABLE 1**

| Test compound | IC₅₀ (µM) |
|---|---|
| Paroxetine (reference compound) | 4.6 |
| Compound A | 0.27 |

### [Example 2]

Proliferation of spinal microglial cells and increasing of expression of P2X₄ receptor in the acute phase of autoimmune neuritis were researched by immunohistological analysis using the EAN rat (Beiter et al.: J. Neuro-immunol. 2005 Mar.; 160(1-2):25-31).

### ((Experimental procedure)

Nine-week-old male LEW/CrlCrlj rat was anesthetized with isoflurane, and an adjuvant or P2 peptide-adjuvant solution was administered by intradermal tale base injection in an amount of 80 µg/80 µL/rat to obtain the EAN rat model. The P2 peptide-adjuvant solution was prepared by dissolving neuritogenic P2 peptide of peripheral myelin (amino acids 53-78: TESPFKNTEISFKLGQEFEETTADNR) in PBS, and mixing the obtained 2 mg/mL solution with complete Freund's adjuvant containing 2 mg/mL (the same concentration) of mycobacterium tuberculosis.

Eighteen days after immunization, the spinal cord was collected after perfusion of 4% neutral buffered paraformaldehyde, embedded with paraffin to prepare slices. A specimen in cross section was prepared at the fifth lumbar level (L5) of the spinal cord, and was subjected to an immunohistological staining using Iba1 antibody, which has widely been used as a microglia marker, and P2X₄ receptor antibody.

### (Experimental results)

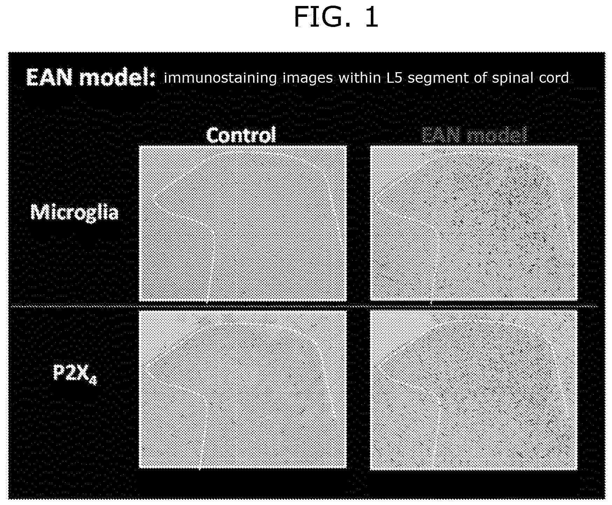

The obtained immunostaining images are shown in Fig. 1. It is observed that Ibal (antigen specific to microglia)-positive cell signals (upper figures) and P2X₄ receptor-positive signals (lower figures) increase within L5 or the spinal cord, compared with the sides administered with only adjuvant.

### [Example 3]

### (Experimental procedure)

Six-week-old male LEW/CrlCrlj rat was acclimatized for about one week, and an indwelling polystyrene catheter with a 0.30 mm outside diameter was placed into the subarachnoid space. Three days or more after indwelling of the catheter for administration into the subarachnoid space, the rat was anesthetized with isoflurane, and an adjuvant or P2 peptide-adjuvant solution was administered by intradermal tale base injection in an amount of 80 µg/80 µL/rat. The compound A was continuously administered by Micro Infusion Pump (Primetech). The pump was placed at the same time of administration of P2 peptide-adjuvant. Administration of the compound A solution was started while placing the pump. After immunization, change of pain threshold was observed with the passage of time.

### (Experimental results)

Fig. 2 shows influence of preventive administration of the compound A on pain threshold of EAN rat model. The animal was administered with P2 peptide, and neuritis associated with paresis of hind legs was observed about ten days after immunization. Further, allodynia was simultaneously observed. Thereafter, allodynia was continued for about 50 days. The animal model was preventively administered with the compound A to suppress pains in initial and later manifestations of the disease.

### [Example 4]

### (Experimental procedure)

Six-week-old male LEW/CrlCrlj rat was acclimatized for about one week, and an indwelling polystyrene catheter with a 0.30 mm outside diameter was placed into the subarachnoid space. Three days or more after indwelling of the catheter for administration into the subarachnoid space, the rat was anesthetized with isoflurane, and an adjuvant or P2 peptide-adjuvant solution was administered by intradermal tale base injection in an amount of 80 µg/80 µL/rat. Micro Infusion Pump was simultaneously placed into the back of the rat, and administration of the vehicle into the subarachnoid space was initiated. After immunization, symptom was observed (Table 2), and change of pain threshold was observed. Thirteen days after immunization, the average of manifestation scores rose up to 2 or more, and they were divided into groups to observe influence of therapeutic administration of the compound A on pain threshold.

### (Experimental results)

Fig. 3 shows influence of therapeutic administration of the compound A on pain threshold of EAN rat model.

A significant analgesic effect on pain in later manifestation of the disease was observed in therapeutic administration as well as the preventive administration.

**TABLE 2**

| Scores of symptom observation | |
|---|---|
| Score 0 | Normal |
| Score 1 | Reduced tone of the tail |
| Score 2 | Limp tail |
| Score 3 | Gate ataxia. |
| Score 4 | Hemiplegia of the hind leg |
| Score 5 | Paraplegia of the hind legs |
| Score 6 | Tetraparesis |
| Score 7 | Moribond |
| Score 8 | Death |

## Claims

1. A compound having the following formula (IX) or a pharmacologically acceptable salt thereof for use in the prevention or treatment of neuropathic pain associated with Guillain-Barré syndrome: 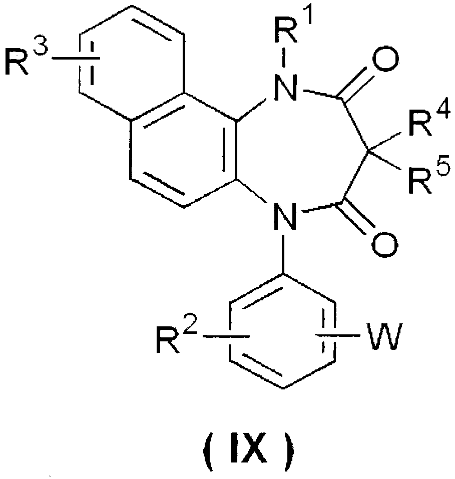
wherein R¹ is hydrogen, a C₁₋₈ alkyl group, a C₂₋₈ alkenyl group, a C₁₋₈ alkyl group having one to three halogen atoms, or a C₁₋₃ alkyl group having phenyl;
each of R² and R³ independently is hydrogen, a C₁₋₈ alkyl group, a C₁₋₈ alkoxy group, a C₁₋₈ alkyl group having one to three halogen atoms, a C₁₋₈ alkoxy group having one to three halogen atoms, a halogen atom, hydroxyl, nitro, cyano, amino, a C₁₋₈ alkylamino group, a C₂₋₈ dialkylamino group, a C₂₋₈ acylamino group, a C₂₋₈ acylamino group having one to three halogen atoms, a C₁₋₈ alkylsulfonylamino group, carboxyl, a C₂₋₈ acyl group, an alkoxycarbonyl group comprising a C₁₋₈ alkoxy moiety, carbamoyl, a C₁₋₈ alkylthio group, a C₁₋₈ alkylsulfinyl group, a C₁₋₈ alkylsulfonyl group, or sulfamoyl;
each of R⁴ and R⁵ independently is hydrogen, a C₁₋₈ alkyl group, a C₁₋₈ alkyl group having one to three halogen atoms, or a C₁₋₃ alkyl group having phenyl; and
W is a five-membered or six-membered heterocyclic ring optionally having one or more substituents and comprising one to four nitrogen atoms as the members of the ring.

2. A compound having the formula (IX) described in claim 1 or a pharmacologically acceptable salt thereof for use in the prevention or treatment of neuropathic pain associated with Guillain-Barré syndrome,
wherein W is tetrazole, 1,2,4-triazole, 1,2,3-triazole, 1,2,4-oxadiazole, pyrazole, or imidazole, each of which optionally has one or more substituents selected from the group consisting of a C₁₋₈ alkyl group, a C₁₋₈ alkyl group having one to three halogen atoms, a halogen atom, cyano, oxo, and thioxo.

3. A compound having the formula (IX) described in claim 1 or a pharmacologically acceptable salt thereof for use in the prevention or treatment of neuropathic pain associated with Guillain-Barré syndrome,
wherein W is tetrazole.

4. A compound having the formula (IX) described in claim 1 or a pharmacologically acceptable salt thereof for use in the prevention or treatment of neuropathic pain associated with Guillain-Barré syndrome,
wherein W is tetrazole, 1,2,4-triazole, 1,2,3-triazole, 1,2,4-oxadiazole, pyrazole, or imidazole, each of which optionally has one or more substituents selected from the group consisting of a C₁₋₈ alkyl group, a C₁₋₈ alkyl group having one to three halogen atoms, a halogen atom, cyano, oxo, and thioxo; or
wherein W is tetrazole, 1,2,4-triazole, or 1,2,3-triazole, each of which optionally has one or more substituents selected from the group consisting of a C₁₋₈ alkyl group, a C₁₋₈ alkyl group having one to three halogen atoms, a halogen atom, and cyano; or
wherein W is 5-oxo-1,2,4-oxadiazole or 5-thioxo-1,2,4-oxadiazole; or
wherein W is tetrazole; or
wherein R¹ is hydrogen or a C₁₋₈ alkyl group; or wherein R¹ is hydrogen; or
wherein R⁴ is hydrogen, and R⁵ is hydrogen or a C₁₋₈ alkyl group; or
wherein each of R⁴ and R⁵ is hydrogen; or
wherein R² is ydrogen, a C₁₋₈ alkyl group, a C₁₋₈ alkoxy group, a C₁₋₈ alkyl group having one to three halogen atoms, a C₁₋₈ alkoxy group having one to three halogen atoms, a halogen atom, hydroxyl, nitro, cyano, amino, carboxyl, a C₂₋₈ acyl group, or an alkoxycarbonyl group comprising a C₁₋₈ alkoxy moiety; or
wherein R² is hydrogen; or
wherein R³ is hydrogen, a C₁₋₈ alkyl group, a C₁₋₈ alkoxy group, a C₁₋₈ alkyl group having one to three halogen atoms, a C₁₋₈ alkoxy group having one to three halogen atoms, a halogen atom, hydroxyl, nitro, cyano, amino, carboxyl, a C₂₋₈ acyl group, or an alkoxycarbonyl group comprising a C₁₋₈ alkoxy moiety; or
wherein R³ is hydrogen; or
wherein the compound is 5-[3-(1H-tetrazol-5-yl)phenyl]-1H-naphtho[1,2-b][1,4]diazepine-2,4(3H,5H)-dione potassium salt.

5. A compound having the formula (IX) described in claim 1 or a pharmacologically acceptable salt thereof for use in the prevention or treatment of neuropathic pain associated with Guillain-Barré syndrome,
wherein the compound is 5-[3-(1H-tetrazol-5-yl)phenyl]-1H-naphtho[1,2-b][1,4]diazepine-2,4(3H,5H)-dione potassium salt.

6. A compound having the following formula (I) or a pharmacologically acceptable salt thereof for use in the prevention or treatment of neuropathic pain associated with Guillain-Barré syndrome: 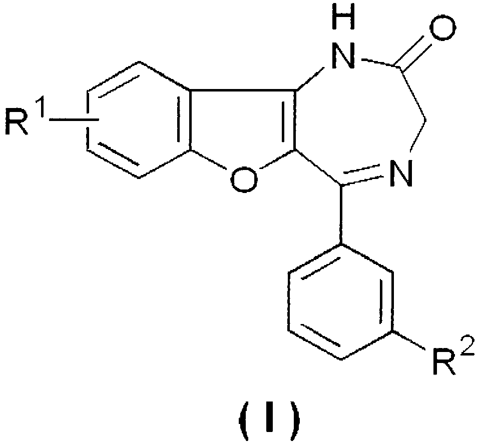
wherein R¹ is a halogen atom; and
R² is hydrogen, a halogen atom, nitro, cyano, -C(O)-OR³, -C(O)-NR⁴R⁵, -SO₂-OR³, or -SO₂-NR⁴R⁵, wherein each of R³, R⁴, and R⁵ is hydrogen or a C₁₋₆ alkyl group; or in the alternative
R¹ is hydrogen; and
R² is a halogen atom, nitro, cyano, -C(O)-OR³, - C(O)-NR⁴R⁵, -SO₂-OR³, or -SO₂-NR⁴R⁵, wherein each of R³, R⁴, and R⁵ is hydrogen or a C₁₋₆ alkyl group.

7. A compound having the following formula (III) or a pharmacologically acceptable salt thereof for use in the prevention or treatment of neuropathic pain associated with Guillain-Barré syndrome: 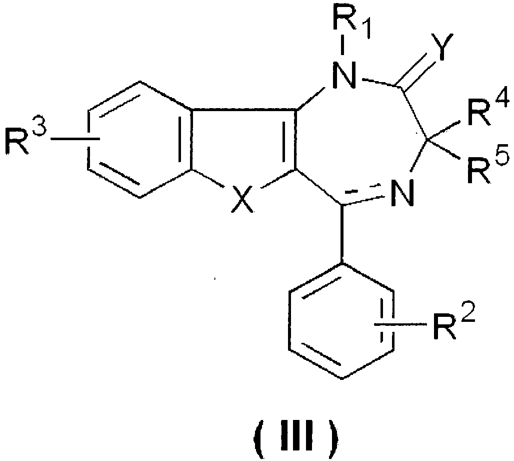
wherein X is S or CH₂;
Y is O, S, or NH;
R¹ is hydrogen, a C₁₋₈ alkyl group, a C₁₋₈ alkyl group having one or more halogen atoms, an aralkyl group comprising a C₁₋₆ alkyl moiety and a C₆₋₁₀ aryl moiety, a C₂₋₈ alkenyl group, carboxymethyl, or an alkoxycarbonylmethyl group comprising a C₁₋₈ alkoxy moiety;
each of R² and R³ independently is hydrogen, a C₁₋₈ alkyl group, a C₁₋₈ alkoxy group, a C₁₋₈ alkyl group having one or more halogen atoms, a C₁₋₈ alkoxy group having one or more halogen atoms, a halogen atom, amino, carboxyl, hydroxyl, nitro, cyano, a C₂₋₈ acyl group, a C₆₋₁₀ aryl group, or a five-membered or six-membered heterocyclic group;
each of R⁴ and R⁵ independently is hydrogen, a C₁₋₈ alkyl group, or a C₁₋₈ alkyl group having one or more halogen atoms; and
the double line consisting of a broken line and a solid line is a single bond or a double bond.

8. A compound having the following formula (IV) or a pharmacologically acceptable salt thereof for use in the prevention or treatment of neuropathic pain associated with Guillain-Barré syndrome: 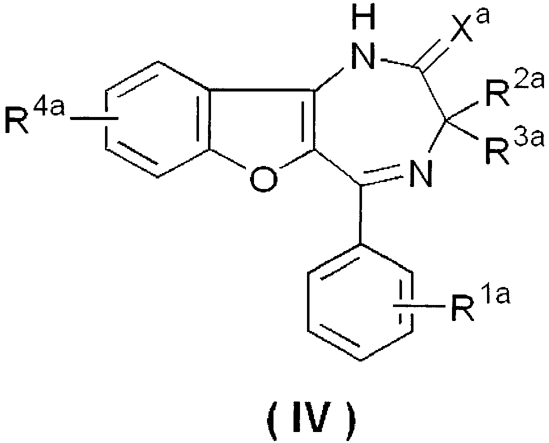
wherein X^{a} is O, S, or NH;
R^{1a} is hydroxyl, tetrazolyl, N(R^{5a})(R^{6a}), a C₂₋₈ alkenyl group, a C₂₋₈ alkynyl group, a C₁₋₈ alkyl group having one or more halogen atoms, a C₁₋₈ alkoxy group having one or more halogen atoms, or a C₆₋₁₀ aryl group, wherein R^{5a} is hydrogen or a C₁₋₈ alkyl group, and R^{6a} is hydrogen, a C₁₋₈ alkyl group, or a C₂₋₈ acyl group;
each of R^{2a} and R^{3a} independently is hydrogen, a C₁₋₈ alkyl group, or a C₁₋₈ alkyl group having one or more halogen atoms; and
R^{4a} is hydrogen, a C₁₋₈ alkyl group, a C₁₋₈ alkoxy group, a C₁₋₈ alkyl group having one or more halogen atoms, a halogen atom, hydroxyl, nitro, amino, carboxyl, tetrazolyl, cyano, a C₆₋₁₀ aryl group, or a five-membered or six-membered heterocyclic group.

9. A compound having the following formula (V) or a pharmacologically acceptable salt thereof for use in the prevention or treatment of neuropathic pain associated with Guillain-Barré syndrome: 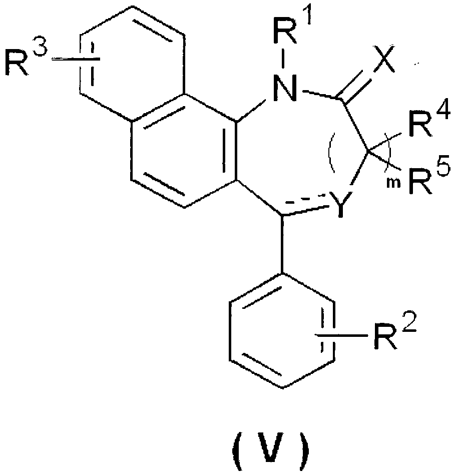
wherein X is O, S, or NH;
Y is N or NR⁶, wherein R⁶ is hydrogen or a C₁₋₈ alkyl group;
R¹ is hydrogen, a C₁₋₈ alkyl group, a C₂₋₈ alkenyl group, a C₁₋₈ alkyl group having one to three halogen atoms, or an alkyl group having phenyl;
R² is a C₁₋₈ alkyl group, a C₁₋₈ alkoxy group, a C₁₋₈ alkyl group having one to three halogen atoms, hydroxyl, nitro, amino, carboxyl, tetrazolyl, or cyano;
R³ is hydrogen, a C₁₋₈ alkyl group, a C₁₋₈ alkoxy group, a C₁₋₈ alkyl group having one to three halogen atoms, a halogen atom, hydroxyl, nitro, amino, carboxyl, tetrazolyl, or cyano;
each of R⁴ and R⁵ independently is hydrogen, a C₁₋₈ alkyl group, or a C₁₋₈ alkyl group having one to three halogen atoms;
m is 1 or 2;
when Y is N, the double line consisting of a solid line and a broken line is a double bond; and
when Y is NR⁶, the double line consisting of a solid line and a broken line is a single bond.

10. A compound having the formula (V) described in claim 9 or a pharmacologically acceptable salt thereof for use in the prevention or treatment of neuropathic pain associated with Guillain-Barré syndrome,
wherein the compound is selected from the group consisting of:
5-(3-methoxyphenyl)-1,3-dihydro-2H-naphtho[1,2-e]-1,4-diazepin-2-one,
5-(3-hydroxyphenyl)-1,3-dihydro-2H-naphtho[1,2-e]-1,4-diazepin-2-one,
5-(4-methoxyphenyl)-1,3-dihydro-2H-naphtho[1,2-e]-1,4-diazepin-2-one,
5-(4-hydroxyphenyl)-1,3-dihydro-2H-naphtho[1,2-e]-1,4-diazepin-2-one,
5-(4-methylphenyl)-1,3-dihydro-2H-naphtho[1,2-e]-1,4-diazepin-2-one,
5-(2-methoxyphenyl)-1,3-dihydro-2H-naphtho[1,2-e]-1,4-diazepin-2-one,
5-(2-hydroxyphenyl)-1,3-dihydro-2H-naphtho[1,2-e]-1,4-diazepin-2-one,
5-(3,4-dimethoxyphenyl)-1,3-dihydro-2H-naphtho[1,2-e]-1,4-diazepin-2-one,and
5-(3,4-dihydroxyphenyl)-1,3-dihydro-2H-naphtho[1,2-e]-1,4-diazepin-2-one.

11. A compound having the following formula (VIII) or a pharmacologically acceptable salt thereof for use in the prevention or treatment of neuropathic pain associated with Guillain-Barré syndrome: 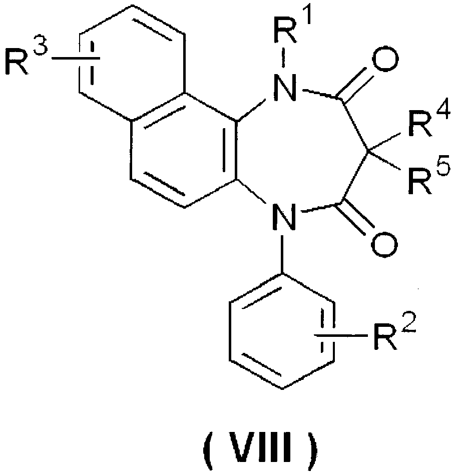
wherein R¹ is hydrogen, a C₁₋₈ alkyl group, a C₂₋₈ alkenyl group, a C₁₋₈ alkyl group having one to three halogen atoms, or a C₁₋₃ alkyl group having phenyl;
R² is hydrogen, a C₁₋₈ alkyl group, a C₁₋₈ alkoxy group, a C₁₋₈ alkyl group having one to three halogen atoms, a C₁₋₈ alkoxy group having one to three halogen atoms, a halogen atom, hydroxyl, nitro, cyano, amino, a C₁₋₈ alkylamino group, a C₂₋₈ dialkylamino group, a C₂₋₈ acylamino group, a C₂₋₈ acylamino group having one to three halogen atoms, a C₁₋₈ alkylsulfonylamino group, carboxyl, a C₂₋₈ acyl group, an alkoxycarbonyl group comprising a C₁₋₈ alkoxy moiety, carbamoyl, a C₁₋₈ alkylthio group, a C₁₋₈ alkylsulfinyl group, a C₁₋₈ alkylsulfonyl group, or sulfamoyl;
R³ is hydrogen, a C₁₋₈ alkyl group, a C₁₋₈ alkoxy group, a C₁₋₈ alkyl group having one to three halogen atoms, a C₁₋₈ alkoxy group having one to three halogen atoms, a halogen atom, hydroxyl, nitro, cyano, amino, carboxyl, a C₂₋₈ acyl group, or an alkoxycarbonyl group comprising a C₁₋₈ alkoxy moiety; and
each of R⁴ and R⁵ independently is hydrogen, a C₁₋₈ alkyl group, or a C₁₋₈ alkyl group having one to three halogen atoms.

12. The compound for use according to claim 11,
wherein R¹ is hydrogen.

13. The compound for use according to claim 11,
wherein R² is a C₂₋₈ acylamino group having one to three halogen atoms.

14. The compound for use according to claim 11,
wherein R³ is hydrogen.

15. The compound for use according to claim 11,
wherein each of R⁴ and R⁵ is hydrogen.

## Patentansprüche

1. Verbindung der nachstehenden Formel (IX) oder ein pharmakologisch annehmbares Salz davon zur Verwendung bei der Vorbeugung oder Behandlung von neuropathischen Schmerzen in Verbindung mit dem Guillain-Barré-Syndrom: 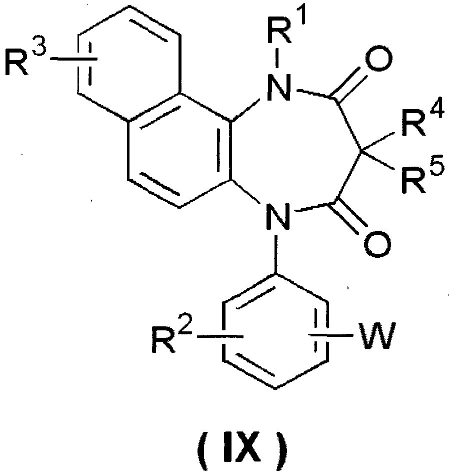
worin R¹ Wasserstoff, eine C₁₋₈-Alkylgruppe, eine C₂₋₈-Alkenylgruppe, eine C₁₋₈-Alkylgrupe mit einem bis drei Halogenatom(en) oder eine C₁₋₃-Alkylgruppe mit Phenyl ist;
jedes von R² und R³ unabhängig Wasserstoff, eine C₁₋₈-Alkylgruppe, eine C₁₋₈-Alkoxygruppe, eine C₁₋₈-Alkylgruppe mit einem bis drei Halogenatom(en), eine C₁₋₈-Alkoxygruppe mit einem bis drei Halogenatom(en), ein Halogenatom, Hydroxyl, Nitro, Cyano, Amino, eine C₁₋₈-Alkylaminogruppe, eine C₂₋₈-Dialkylaminogruppe, eine C₂₋₈-Acylaminogruppe, eine C₂₋₈-Acylaminogruppe mit einem bis drei Halogenatom(en), eine C₁₋₈-Alkylsulfonylaminogruppe, Carboxyl, eine C₂₋₈-Acylgruppe, eine Alkoxycarbonylgruppe, umfassend einen C₁₋₈-Alkoxyrest, Carbamoyl, eine C₁₋₈-Alkylthiogruppe, eine C₁₋₈-Alkylsulfinyl-gruppe, eine C₁₋₈-Alkylsulfonylgruppe oder Sulfamoyl ist;
jedes von R⁴ und R⁵ unabhängig Wasserstoff, eine C₁₋₈-Alkylgruppe, eine C₁₋₈-Alkylgruppe mit einem bis drei Halogenatom(en) oder eine C₁₋₃-Alkylgruppe mit Phenyl ist; und
W ein fünfgliedriger oder sechsgliedriger heterocyclischer Ring ist, gegebenenfalls mit einem oder mehreren Substituenten und umfassend ein bis vier Stickstoffatom(e) als Ringglieder.

2. Verbindung der Formel (IX), beschrieben in Anspruch 1, oder ein pharmakologisch annehmbares Salz davon zur Verwendung bei der Vorbeugung oder Behandlung von neuropathischen Schmerzen in Verbindung mit dem Guillain-Barré-Syndrom, worin W Tetrazol, 1,2,4-Triazol, 1,2,3-Triazol, 1,2,4-Oxadiazol, Pyrazol oder Imidazol ist, wobei jedes davon gegebenenfalls einen oder mehrere Substituenten aufweist, ausgewählt aus der Gruppe, bestehend aus einer C₁₋₈-Alkylgruppe, einer C₁₋₈-Alkylgruppe mit einem bis drei Halogenatomen, einem Halogenatom, Cyano, Oxo und Thioxo.

3. Verbindung der Formel (IX), beschrieben in Anspruch 1, oder ein pharmakologisch annehmbares Salz davon zur Verwendung bei der Vorbeugung oder Behandlung von neuropathischen Schmerzen in Verbindung mit dem Guillain-Barré-Syndrom, worin W Tetrazol ist.

4. Verbindung der Formel (IX), beschrieben in Anspruch 1, oder ein pharmakologisch annehmbares Salz davon zur Verwendung bei der Vorbeugung oder Behandlung von neuropathischen Schmerzen in Verbindung mit dem Guillain-Barre-Syndrom,
worin W Tetrazol, 1,2,4-Triazol, 1,2,3-Triazol, 1,2,4-Oxadiazol, Pyrazol oder Imidazol ist, wobei jedes davon gegebenenfalls einen oder mehrere Substituenten aufweist, ausge-wählt aus der Gruppe, bestehend aus einer C₁₋₈-Alkylgruppe, einer C₁₋₈-Alkylgruppe mit einem bis drei Halogenatom(en), einem Halogenatom, Cyano, Oxo und Thioxo; oder
worin W Tetrazol, 1,2,4-Triazol oder 1,2,3-Triazol ist, wobei jedes davon gegebenenfalls einen oder mehrere Substituenten aufweist, ausgewählt aus der Gruppe, bestehend aus einer C₁₋₈-Alkylgruppe, einer C₁₋₈-Alkylgruppe mit einem bis drei Halogenatom(en), einem Halogenatom und Cyano; oder
worin W 5-Oxo-1,2,4-oxadiazol oder 5-Thioxo-1,2,4-oxadiazol ist; oder
worin W Tetrazol ist; oder
worin R¹ Wasserstoff oder eine C₁₋₈-Alkylgruppe ist; oder worin R¹ Wasserstoff ist; oder
worin R⁴ Wasserstoff ist und R⁵ Wasserstoff oder eine C₁₋₈-Alkylgruppe ist; oder
worin jedes von R⁴ und R⁵ Wasserstoff ist; oder
worin R² Wasserstoff, eine C₁₋₈-Alkylgruppe, eine C₁₋₈-Alkoxygruppe, eine C₁₋₈-Alkylgruppe mit einem bis drei Halogenatom(en), eine C₁₋₈-Alkoxygruppe mit einem bis drei Halogenatom(en), ein Halogenatom, Hydroxyl, Nitro, Cyano, Amino, Carboxyl, eine C₂₋₈-Acylgruppe oder eine Alkoxycarbonylgruppe, umfassend einen C₁₋₈-Alkoxyrest, ist; oder
worin R² Wasserstoff ist; oder
worin R³ Wasserstoff, eine C₁₋₈-Alkylgruppe, eine C₁₋₈-Alkoxygruppe, eine C₁₋₈-Alkylgruppe mit einem bis drei Halogenatom(en), eine C₁₋₈-Alkoxygruppe mit einem bis drei Halogenatom(en), ein Halogenatom, Hydroxyl, Nitro, Cyano, Amino, Carboxyl, eine C₂₋₈-Acylgruppe oder eine Alkoxycarbonylgruppe, umfassend einen C₁₋₈-Alkoxyrest, ist; oder
worin R³ Wasserstoff ist; oder
worin die Verbindung das Kaliumsalz von 5-[3-(1H-Tetrazol-5-yl)phenyl]-1H-naphtho[1,2-b][1,4]diazepin-2,4(3H,5H)-dion ist.

5. Verbindung der Formel (IX), beschrieben in Anspruch 1, oder ein pharmakologisch annehmbares Salz davon zur Verwendung bei der Vorbeugung oder Behandlung von neuropathischen Schmerzen in Verbindung mit dem Guillain-Barre-Syndrom, wobei die Verbindung das Kaliumsalz von 5-[3-(1H-Tetrazol-5-yl)phenyl]-1H-naphtho[1,2-b][1,4]diazepin-2,4(3H,5H)-dion ist.

6. Verbindung der nachstehenden Formel (I) oder ein pharmakologisch annehmbares Salz davon zur Verwendung bei der Vorbeugung oder Behandlung von neuropathischen Schmerzen in Verbindung mit dem Guillain-Barré-Syndrom: 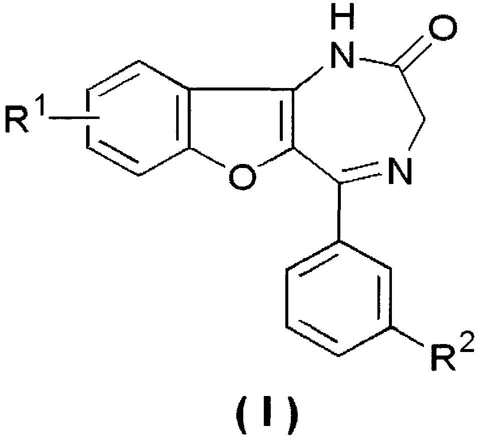
worin R¹ ein Halogenatom ist; und
R² Wasserstoff, ein Halogenatom, Nitro, Cyano, -C(O)-OR³, -C(O)-NR⁴R⁵, -SO₂-OR³ oder -SO₂-NR⁴R⁵ ist, wobei jedes von R³, R⁴ und R⁵ Wasserstoff oder eine C₁₋₆-Alkylgruppe ist; oder alternativ
R¹ Wasserstoff ist; und
R² ein Halogenatom, Nitro, Cyano, -C(O)-OR³ , -C(O)-NR⁹R⁵ , -SO₂-OR³ oder -SO₂-NR⁴R⁵ ist, wobei jedes von R³, R⁴ und R⁵ Wasserstoff oder eine C₁₋₆-Alkylgruppe ist.

7. Verbindung der nachstehenden Formel (III) oder ein pharmakologisch annehmbares Salz davon zur Verwendung bei der Vorbeugung oder Behandlung von neuropathischen Schmerzen in Verbindung mit dem Guillain-Barré-Syndrom: 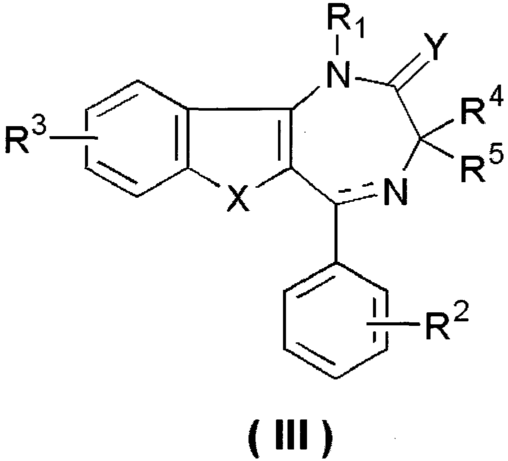
worin X S oder CH₂ ist;
Y O, S oder NH ist;
R¹ Wasserstoff, eine C₁₋₈-Alkylgruppe, eine C₁₋₈-Alkylgruppe mit einem oder mehreren Halogenatom(en), eine Aralkylgruppe, umfassend einen C₁₋₆-Alkylrest und einen C₆₋₁₀-Arylrest, eine C₂₋₈-Alkenylgruppe, Carboxymethyl oder eine Alkoxycarbonylmethylgruppe, umfassend einen C₁₋₈-Alkoxyrest, ist;
jedes von R² und R³ unabhängig Wasserstoff, eine C₁₋₈-Alkylgruppe, eine C₁₋₈-Alkoxygruppe, eine C₁₋₈-Alkylgruppe mit einem oder mehreren Halogenatom(en), eine C₁₋₈-Alkoxygruppe mit einem oder mehreren Halogenatom(en), ein Halogenatom, Amino, Carboxyl, Hydroxyl, Nitro, Cyano, eine C₂₋₈-Acylgruppe, eine C₆₋₁₀-Arylgruppe oder eine fünfgliedrige oder sechsgliedrige heterocyclische Gruppe ist;
jedes von R⁴ und R⁵ unabhängig Wasserstoff, eine C₁₋₈-Alkylgruppe oder eine C₁₋₈-Alkylgruppe mit einem oder mehreren Halogenatom(en) ist; und
die Doppellinie, bestehend aus einer unterbrochenen Linie und einer durchgehenden Linie, eine Einfachbindung oder eine Doppelbindung ist.

8. Verbindung der nachstehenden Formel (IV) oder ein pharmakologisch annehmbares Salz davon zur Verwendung bei der Vorbeugung oder Behandlung von neuropathischen Schmerzen in Verbindung mit dem Guillain-Barré-Syndrom: 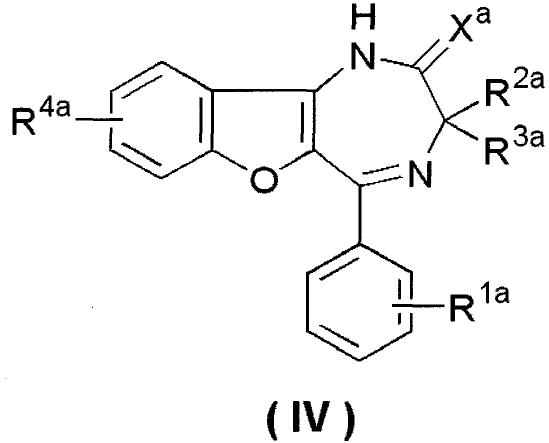
worin X^{a} O, S oder NH ist;
R^{1a} Hydroxyl, Tetrazolyl, N(R^{5a})(R^{6a}), eine C₂₋₈-Alkenylgruppe, eine C₂₋₈-Alkinylgruppe, eine C₁₋₈-Alkylgruppe mit einem oder mehreren Halogenatom(en), eine C₁₋₈-Alkoxygruppe mit einem oder mehreren Halogenatom(en) oder eine C₆₋₁₀-Arylgruppe ist,
worin R^{5a} Wasserstoff oder eine C₁₋₈-Alkylgruppe ist und R^{6a} Wasserstoff, eine C₁₋₈-Alkylgruppe oder eine C₂₋₈-Acylgruppe ist;
jedes von R^{2a} und R^{3a} unabhängig Wasserstoff, eine C₁₋₈-Alkylgruppe oder eine C₁₋₈-Alkylgruppe mit einem oder mehreren Halogenatom(en) ist; und
R^{4a} Wasserstoff, eine C₁₋₈-Alkylgruppe, eine C₁₋₈-Alkoxygruppe, eine C₁₋₈-Alkylgruppe mit einem oder mehreren Halogenatom(en), ein Halogenatom, Hydroxyl, Nitro, Amino, Carboxyl, Tetrazolyl, Cyano, eine C₆₋₁₀-Arylgruppe oder eine fünfgliedrige oder sechsgliedrige heterocyclische Gruppe ist.

9. Verbindung der nachstehenden Formel (V) oder ein pharmakologisch annehmbares Salz davon zur Verwendung bei der Vorbeugung oder Behandlung von neuropathischen Schmerzen in Verbindung mit dem Guillain-Barré-Syndrom: 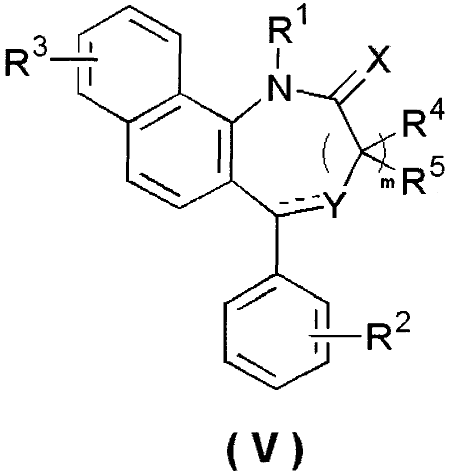
worin X O, S oder NH ist;
Y N oder NR⁶ ist, wobei R⁶ Wasserstoff oder eine C₁₋₈-Alkylgruppe ist;
R¹ Wasserstoff, eine C₁₋₈-Alkylgruppe, eine C₂₋₈-Alkenylgruppe, eine C₁₋₈-Alkylgruppe mit einem bis drei Halogenatom(en) oder eine Alkylgruppe mit Phenyl ist;
R² eine C₁₋₈-Alkylgruppe, eine C₁₋₈-Alkoxygruppe, eine C₁₋₈-Alkylgruppe mit einem bis drei Halogenatom(en), Hydroxyl, Nitro, Amino, Carboxyl, Tetrazolyl oder Cyano ist;
R³ Wasserstoff, eine C₁₋₈-Alkylgruppe, eine C₁₋₈-Alkoxygruppe, eine C₁₋₈-Alkylgruppe mit einem bis drei Halogenatom(en), ein Halogenatom, Hydroxyl, Nitro, Amino, Carboxyl, Tetrazolyl oder Cyano ist;
jedes von R⁴ und R⁵ unabhängig Wasserstoff, eine C₁₋₈-Alkylgruppe oder eine C₁₋₈-Alkylgruppe mit einem bis drei Halogenatom(en) ist;
m 1 oder 2 ist;
wenn Y N ist, die Doppellinie, bestehend aus einer durchgehenden Linie und einer unterbrochenen Linie, eine Doppelbindung ist; und
wenn Y NR⁶ ist, die Doppellinie, bestehend aus einer durchgehenden Linie und einer unterbrochenen Linie, eine Einfachbindung ist.

10. Verbindung der Formel (V), beschrieben in Anspruch 9, oder ein pharmakologisch annehmbares Salz davon zur Verwendung bei der Vorbeugung oder Behandlung von neuropathischen Schmerzen in Verbindung mit dem Guillain-Barré-Syndrom, wobei die Verbindung ausgewählt ist aus der Gruppe, bestehend aus:
5-(3-Methoxyphenyl)-1,3-dihydro-2H-naphtho[1,2-e]-1,4-diazepin-2-on,
5-(3-Hydroxyphenyl)-1,3-dihydro-2H-naphtho[1,2-e]-1,4-diazepin-2-on,
5-(4-Methoxyphenyl)-1,3-dihydro-2H-naphtho[1,2-e]-1,4-diazepin-2-on,
5-(4-Hydroxyphenyl)-1,3-dihydro-2H-naphtho[1,2-e]-1,4-diazepin-2-on,
5-(4-Methylphenyl)-1,3-dihydro-2H-naphtho[1,2-e]-1,4-diazepin-2-on,
5-(2-Methoxyphenyl)-1,3-dihydro-2H-naphtho[1,2-e]-1,4-diazepin-2-on,
5-(2-Hydroxyphenyl)-1,3-dihydro-2H-naphtho[1,2-e]-1,4-diazepin-2-on,
5-(3,4-Dimethoxyphenyl)-1,3-dihydro-2H-naphtho[1,2-e]-1,4-diazepin-2-on und
5-(3,4-Dihydroxyphenyl)-1,3-dihydro-2H-naphtho[1,2-e]-1,4-diazepin-2-on.

11. Verbindung der nachstehenden Formel (VIII) oder ein pharmakologisch annehmbares Salz davon zur Verwendung bei der Vorbeugung oder Behandlung von neuropathischen Schmerzen in Verbindung mit dem Guillain-Barré-Syndrom: 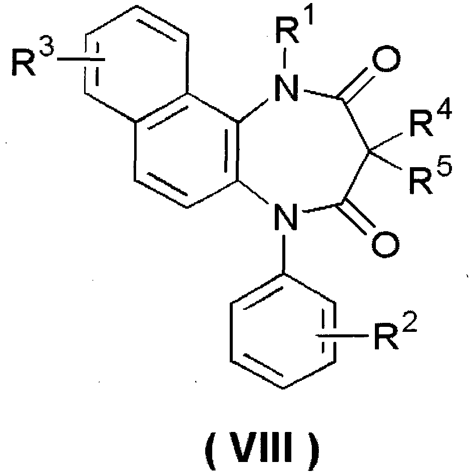
worin R¹ Wasserstoff, eine C₁₋₈-Alkylgruppe, eine C₂₋₈-Alkenylgruppe, eine C₁₋₈-Alkylgruppe mit einem bis drei Halogenatom(en) oder eine C₁₋₃-Alkylgruppe mit Phenyl ist;
R² Wasserstoff, eine C₁₋₈-Alkylgruppe, eine C₁₋₈-Alkoxygruppe, eine C₁₋₈-Alkylgruppe mit einem bis drei Halogenatom(en), eine C₁₋₈-Alkoxygruppe mit einem bis drei Halogenatom(en), ein Halogenatom, Hydroxyl, Nitro, Cyano, Amino, eine C₁₋₈-Alkylaminogruppe, eine C₂₋₈-Dialkylaminogruppe, eine C₂₋₈-Acylaminogruppe, eine C₂₋₈-Acylaminogruppe mit einem bis drei Halogenatom(en), eine C₁₋₈-Alkylsulfonylaminogruppe, Carboxyl, eine C₂₋₈-Acylgruppe, eine Alkoxycarbonylgruppe, umfassend einen C₁₋₈-Alkoxyrest, Carbamoyl, eine C₁₋₈-Alkylthiogruppe, eine C₁₋₈-Alkylsulfinylgruppe, eine C₁₋₈-Alkylsulfonylgruppe oder Sulfamoyl ist;
R³ Wasserstoff, eine C₁₋₈-Alkylgruppe, eine C₁₋₈-Alkoxygruppe, eine C₁₋₈-Alkylgruppe mit einem bis drei Halogenatom(en), eine C₁₋₈-Alkoxygruppe mit einem bis drei Halogenatom(en), ein Halogenatom, Hydroxyl, Nitro, Cyano, Amino, Carboxyl, eine C₂₋₈-Acylgruppe oder eine Alkoxycarbonylgruppe, umfassend einen C₁₋₈-Alkoxyrest, ist; und
jedes von R⁴ und R⁵ unabhängig Wasserstoff, eine C₁₋₈-Alkylgruppe oder eine C₁₋₈-Alkylgruppe mit einem bis drei Halogenatom(en) ist.

12. Verbindung zur Verwendung gemäß Anspruch 11,
worin R¹ Wasserstoff ist.

13. Verbindung zur Verwendung gemäß Anspruch 11,
worin R² eine C₂₋₈-Acylaminogruppe mit einem bis drei Halogenatom(en) ist.

14. Verbindung zur Verwendung gemäß Anspruch 11,
worin R³ Wasserstoff ist.

15. Verbindung zur Verwendung gemäß Anspruch 11,
worin jedes von R⁴ und R⁵ Wasserstoff ist.

## Revendications

1. Composé ayant la formule (IX) suivante ou sel pharmacologiquement acceptable de celui-ci pour une utilisation dans la prévention ou le traitement d'une douleur neuropathique associée au syndrome de Guillain-Barré : 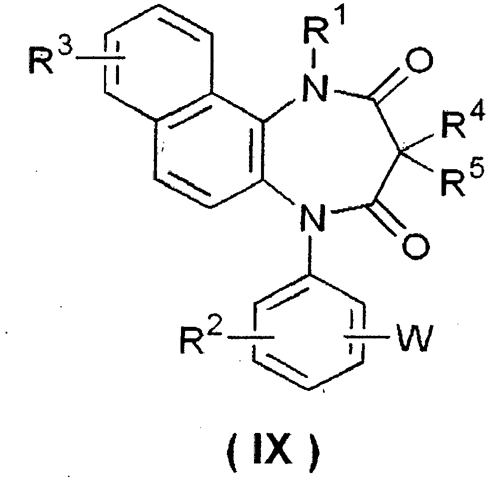
dans lequel R¹ est hydrogène, un groupe alkyle en C_{1 à 8}, un groupe alcényle en C_{2 à 8}, un groupe alkyle en C_{1 à 8} comportant un à trois atomes d'halogène ou un groupe alkyle en C_{1 à 3} comportant un phényle ;
chacun de R² et R³ indépendamment est hydrogène, un groupe alkyle en C_{1 à 8}, un groupe alcoxy en C_{1 à 8}, un groupe alkyle en C_{1 à 8} comportant un à trois atomes d'halogène, un groupe alcoxy en C_{1 à 8} comportant un à trois atomes d'halogène, un atome d'halogène, hydroxyle, nitro, cyano, amino, un groupe alkylamino en C_{1 à 8}, un groupe dialkylamino en C_{2 à 8}, un groupe acylamino en C_{2 à 8}, un groupe acylamino en C_{2 à 8} comportant un à trois atomes d'halogène, un groupe alkylsulfonylamino en C_{1 à 8}, carboxyle, un groupe acyle en C_{2 à 8}, un groupe alcoxycarbonyle comprenant une fraction alcoxy en C_{1 à 8}, carbamoyle, un groupe alkylthio en C_{1 à 8}, un groupe alkylsulfinyle en C_{1 à 8}, un groupe alkylsulfonyle en C_{1 à 8}, ou sulfamoyle ;
chacun de R⁴ et R⁵ indépendamment est hydrogène, un groupe alkyle en C_{1 à 8}, un groupe alkyle en C_{1 à 8} comportant un à trois atomes d'halogène ou un groupe alkyle en C_{1 à 3} comportant un phényle ; et
W est un cycle hétérocyclique à cinq chaînons ou six chaînons comportant facultativement un ou plusieurs substituants et comprenant un à quatre atomes d'azote en tant que membres du cycle.

2. Composé ayant la formule (IX) décrite dans la revendication 1 ou sel pharmacologiquement acceptable de celui-ci pour une utilisation dans la prévention ou le traitement d'une douleur neuropathique associée au syndrome de Guillain-Barré,
dans lequel W est tétrazole, 1,2,4-triazole, 1,2,3-triazole, 1,2-4-oxadiazole, pyrazole ou imidazole, dont chacun comporte facultativement un ou plusieurs substituants sélectionnés dans le groupe consistant en un groupe alkyle en C_{1 à 8}, un groupe alkyle en C_{1 à 8} comportant un à trois atomes d'halogène, un atome d'halogène, cyano, oxo et thioxo.

3. Composé ayant la formule (IX) décrite dans la revendication 1 ou sel pharmacologiquement acceptable de celui-ci pour une utilisation dans la prévention ou le traitement d'une douleur neuropathique associée au syndrome de Guillain-Barré,
dans lequel W est tétrazole.

4. Composé ayant la formule (IX) décrite dans la revendication 1 ou sel pharmacologiquement acceptable de celui-ci pour une utilisation dans la prévention ou le traitement d'une douleur neuropathique associée au syndrome de Guillain-Barré,
dans lequel W est tétrazole, 1, 2, 4-triazole, 1,2,3-triazole, 1,2-4-oxadiazole, pyrazole ou imidazole, dont chacun comporte facultativement un ou plusieurs substituants sélectionnés dans le groupe consistant en un groupe alkyle en C_{1 à 8}, un groupe alkyle en C_{1 à 8} comportant un à trois atomes d'halogène, un atome d'halogène, cyano, oxo et thioxo ; ou
dans lequel W est tétrazole, 1,2,4-triazole ou 1,2,3-triazole, dont chacun comporte facultativement un ou plusieurs substituants sélectionnés dans le groupe consistant en un groupe alkyle en C_{1 à 8}, un groupe alkyle en C_{1 à 8} comportant un à trois atomes d'halogène, un atome d'halogène, et cyano ; ou
dans lequel W est 5-oxo-1,2,4-oxadiazole ou 5-thioxo-1,2,4-oxadiazole ; ou
dans lequel W est tétrazole ; ou
dans lequel R¹ est hydrogène ou un groupe alkyle en C_{1 à 8} ; ou
dans lequel R¹ est hydrogène ; ou
dans lequel R⁴ est hydrogène, et R⁵ est hydrogène ou un groupe alkyle en C_{1 à 8} ; ou
dans lequel chacun de R⁴ et R⁵ est hydrogène ; ou
dans lequel R² est hydrogène, un groupe alkyle en C_{1 à 8}, un groupe alcoxy en C_{1 à 8}, un groupe alkyle en C_{1 à 8} comportant un à trois atomes d'halogène, un groupe alcoxy en C_{1 à 8} comportant un à trois atomes d' halogène, un atome d'halogène, hydroxyle, nitro, cyano, amino, carboxyle, un groupe acyle en C_{2 à 8}, ou un groupe alcoxycarbonyle comprenant une fraction alcoxy en C_{1 à 8} ; ou
dans lequel R² est hydrogène ; ou
dans lequel R³ est hydrogène, un groupe alkyle en C_{1 à 8}, un groupe alcoxy en C_{1 à 8}, un groupe alkyle en C_{1 à 8} comportant un à trois atomes d'halogène, un groupe alcoxy en C_{1 à 8} comportant un à trois atomes d'halogène, un atome d'halogène, hydroxyle, nitro, cyano, amino, carboxyle, un groupe acyle en C_{2 à 8}, ou un groupe alcoxycarbonyle comprenant une fraction alcoxy en C_{1 à 8} ; ou
dans lequel R³ est hydrogène ; ou
dans lequel le composé est le sel de potassium de 5-[3-(1H-tétrazol-5-yl)phényl]-1H-naphto[1,2-b][1,4]diazépine-2,4(3H,5H)-dione.

5. Composé ayant la formule (IX) décrite dans la revendication 1 ou sel pharmacologiquement acceptable de celui-ci pour une utilisation dans la prévention ou le traitement d'une douleur neuropathique associée au syndrome de Guillain-Barré,
dans lequel le composé est le sel de potassium de 5-[3-(1H-tétrazol-5-yl)phényl]-1H-naphto[1,2-b][1,4]diazépine-2,4(3H,5H)-dione.

6. Composé ayant la formule (I) suivante ou sel pharmacologiquement acceptable de celui-ci pour une utilisation dans la prévention ou le traitement d'une douleur neuropathique associée au syndrome de Guillain-Barré : 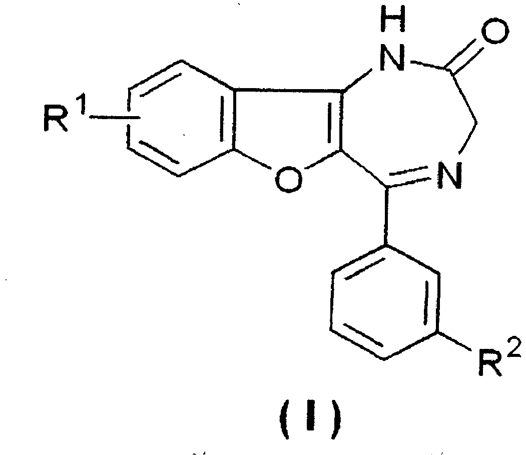
dans lequel R¹ est un atome d'halogène ; et
R² est hydrogène, un atome d'halogène, nitro, cyano, -C(O)-OR³, -C (O) -NR⁴R⁵, -SO₂-OR³ ou -SO₂-NR⁴R⁵, dans lequel chacun de R³, R⁴ et R⁵ est hydrogène ou un groupe alkyle en C_{1 à 6} ; ou en variante
R¹ est hydrogène ; et
R² est un atome d'halogène, nitro, cyano, -C(O)-OR³, -C(O)-NR⁴R⁵, -SO₂-OR³ ou -SO₂-NR⁹R⁵, dans lequel chacun de R³, R⁴ et R⁵ est hydrogène ou un groupe alkyle en C_{1 à 6}.

7. Composé ayant la formule (III) suivante ou sel pharmacologiquement acceptable de celui-ci pour une utilisation dans la prévention ou le traitement d'une douleur neuropathique associée au syndrome de Guillain-Barré : 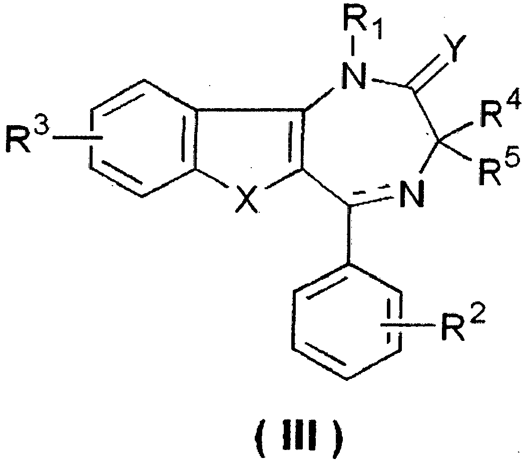
dans lequel X est S ou CH₂ ;
Y est O, S ou NH;
R¹ est hydrogène, un groupe alkyle en C_{1 à 8}, un groupe alkyle en C_{1 à 8} comportant un ou plusieurs atomes d'halogène, un groupe aralkyle comprenant une fraction alkyle en C_{1 à 6} et une fraction aryle en C_{6 à 10}, un groupe alcényle en C_{2 à 8}, carboxyméthyle, ou un groupe alcoxycarbonylméthyle comprenant une fraction alcoxy en C_{1 à 8} ;
chacun de R² et R³ indépendamment est hydrogène, un groupe alkyle en C_{1 à 8}, un groupe alcoxy en C_{1 à 8}, un groupe alkyle en C_{1 à 8} comportant un ou plusieurs atomes d'halogène, un groupe alcoxy en C_{1 à 8} comportant un ou plusieurs atomes d'halogène, un atome d'halogène, amino, carboxyle, hydroxyle, nitro, cyano, un groupe acyle en C_{2 à 8}, un groupe aryle en C_{6 à 10}, ou un groupe hétérocyclique à cinq chaînons ou six chaînons ;
chacun de R⁴ et R⁵ indépendamment est hydrogène, un groupe alkyle en C_{1 à 8}, ou un groupe alkyle en C_{1 à 8} comportant un ou plusieurs atomes d'halogène ; et
la double ligne consistant en une ligne brisée et une ligne pleine est une simple liaison ou une double liaison.

8. Composé ayant la formule (IV) suivante ou sel pharmacologiquement acceptable de celui-ci pour une utilisation dans la prévention ou le traitement d'une douleur neuropathique associée au syndrome de Guillain-Barré : 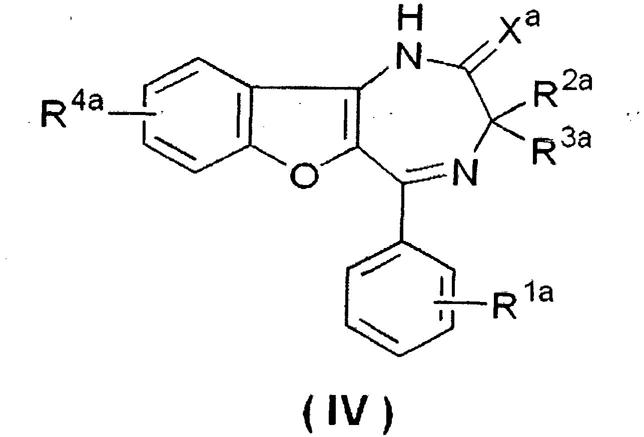
dans lequel X^{a} est O, S ou NH ;
R^{1a} est hydroxyle, tétrazolyle, N(R^{5a})(R^{6a}), un groupe alcényle en C_{2 à 8}, un groupe alcynyle en C_{2 à 8}, un groupe alkyle en C_{1 à 8} comportant un ou plusieurs atomes d'halogène, un groupe alcoxy en C_{1 à 8} comportant un ou plusieurs atomes d'halogène, ou un groupe aryle en C_{6 à 10}, dans lequel R^{5a} est hydrogène ou un groupe alkyle en C_{1 à 8}, et R^{6a} est hydrogène, un groupe alkyle en C_{1 à 8} ou un groupe acyle en C_{2 à 8} ;
chacun de R^{2a} et R^{3a} indépendamment est hydrogène, un groupe alkyle en C_{1 à 8}, ou un groupe alkyle en C_{1 à 8} comportant un ou plusieurs atomes d'halogène ; et
R^{4a} est hydrogène, un groupe alkyle en C_{1 à 8}, un groupe alcoxy en C_{1 à 8}, un groupe alkyle en C_{1 à 8} comportant un ou plusieurs atomes d'halogène, un atome d'halogène, hydroxyle, nitro, amino, carboxyle, tétrazolyle, cyano, un groupe aryle en C_{6 à 10}, ou un groupe hétérocyclique à cinq chaînons ou six chaînons.

9. Composé ayant la formule (V) suivante ou sel pharmacologiquement acceptable de celui-ci pour une utilisation dans la prévention ou le traitement d'une douleur neuropathique associée au syndrome de Guillain-Barré : 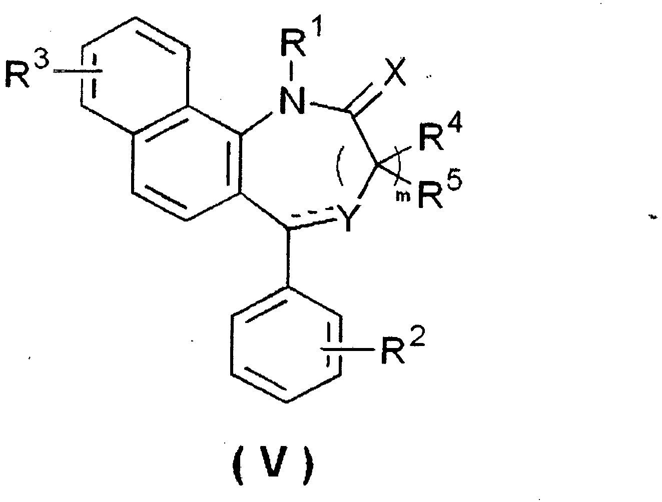
dans lequel X est O, S ou NH ;
Y est N ou NR⁶, dans lequel R⁶ est hydrogène ou un groupe alkyle en C_{1 à 8} ;
R¹ est hydrogène, un groupe alkyle en C_{1 à 8}, un groupe alcényle en C_{2 à 8}, un groupe alkyle en C_{1 à 8} comportant un à trois atomes d'halogène, ou un groupe alkyle comportant un phényle ;
R² est un groupe alkyl en C_{1 à 8}, un groupe alcoxy en C_{1 à 8}, un groupe alkyle en C_{1 à 8} comportant un à trois atomes d'halogène, hydroxyle, nitro, amino, carboxyle, tétrazolyle ou cyano ;
R³ est hydrogène, un groupe alkyle en C_{1 à 8}, un groupe alcoxy en C_{1 à 8}, un groupe alkyle en C_{1 à 8} comportant un à trois atomes d'halogène, un atome d'halogène, hydroxyle, nitro, amino, carboxyle, tétrazolyle ou cyano ;
chacun de R⁴ et R⁵ indépendamment est hydrogène, un groupe alkyle en C_{1 à 8}, ou un groupe alkyle en C_{1 à 8} comportant un à trois atomes d'halogène ;
m est 1 ou 2 ;
lorsqu'Y est N, la double ligne consistant en une ligne pleine et une ligne brisée est une double liaison ; et
lorsqu'Y est NR⁶, la double ligne consistant en une ligne pleine et une ligne brisée est une simple liaison.

10. Composé ayant la formule (V) décrite dans la revendication 9 ou sel pharmacologiquement acceptable de celui-ci pour une utilisation dans la prévention ou le traitement d'une douleur neuropathique associée au syndrome de Guillain-Barré,
dans lequel le composé est sélectionné dans le groupe consistant en :
la 5-(3-méthoxyphényl)-1,3-dihydro-2H-naphto[1,2-e]-1,4-diazépin-2-one,
la 5-(3-hydroxyphényl)-1,3-dihydro-2H-naphto[1,2-e]-1,4-diazépin-2-one,
la 5-(4-méthoxyphényl)-1,3-dihydro-2H-naphto[1,2-e]-1,4-diazépin-2-one,
la 5-(4-hydroxyphényl)-1,3-dihydro-2H-naphto[1,2-e]-1,4-diazépin-2-one,
la 5-(4-méthylphényl)-1,3-dihydro-2H-naphto[1,2-e]-1,4-diazépin-2-one,
la 5-(4-méthoxyphényl)-1,3-dihydro-2H-naphto[1,2-e]-1,4-diazépin-2-one,
la 5-(2-hydroxyphényl)-1,3-dihydro-2H-naphto[1,2-e]-1,4-diazépin-2-one,
la 5-(3,4-diméthoxyphényl)-1,3-dihydro-2H-naphto[1,2-e]-1,4-diazépin-2-one, et
la 5-(3,4-dihydroxyphényl)-1,3-dihydro-2H-naphto[1,2-e]-1,4-diazépin-2-one.

11. Composé ayant la formule (VIII) suivante ou sel pharmacologiquement acceptable de celui-ci pour une utilisation dans la prévention ou le traitement d'une douleur neuropathique associée au syndrome de Guillain-Barré : 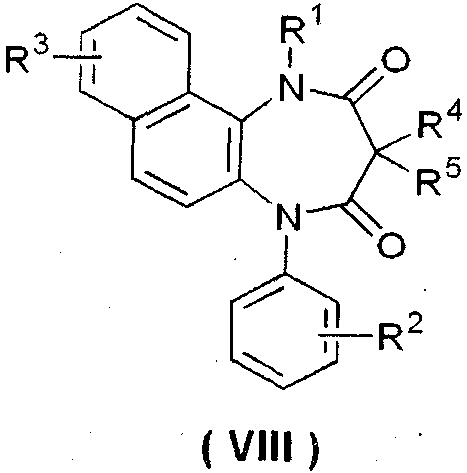
dans lequel R¹ est hydrogène, un groupe alkyle en C_{1 à 8}, un groupe alcényle en C_{2 à 8}, un groupe alkyle en C_{1 à 8} comportant un à trois atomes d'halogène ou un groupe alkyle en C_{1 à 3} comportant un phényle ;
R² est hydrogène, un groupe alkyle en C_{1 à 8}, un groupe alcoxy en C_{1 à 8}, un groupe alkyle en C_{1 à 8} comportant un à trois atomes d'halogène, un groupe alcoxy en C_{1 à 8} comportant un à trois atomes d'halogène, un atome d'halogène, hydroxyle, nitro, cyano, amino, un groupe alkylamino en C_{1 à 8}, un groupe dialkylamino en C_{2 à 8}, un groupe acylamino en C_{2 à 8}, un groupe acylamino en C_{2 à 8} comportant un à trois atomes d'halogène, un groupe alkylsulfonylamino en C_{1 à 8}, carboxyle, un groupe acyle en C_{2 à 8}, un groupe alcoxycarbonyle comprenant une fraction alcoxy en C_{1 à 8}, carbamoyle, un groupe alkylthio en C_{1 à 8}, un groupe alkylsulfinyle en C_{1 à 8}, un groupe alkylsulfonyle en C_{1 à 8}, ou sulfamoyle ;
R³ est hydrogène, un groupe alkyle en C_{1 à 8}, un groupe alcoxy en C_{1 à 8}, un groupe alkyle en C_{1 à 8} comportant un à trois atomes d'halogène, un groupe alcoxy en C_{1 à 8} comportant un à trois atomes d'halogène, un atome d'halogène, hydroxyle, nitro, cyano, amino, carboxyle, un groupe acyle en C_{2 à 8}, ou un groupe alcoxycarbonyle comprenant une fraction alcoxy en C_{1 à 8} ; et
chacun de R⁴ et R⁵ indépendamment est hydrogène, un groupe alkyle en C_{1 à 8} ou un groupe alkyle en C_{1 à 8} comportant un à trois atomes d'halogène.

12. Composé pour une utilisation selon la revendication 11,
dans lequel R¹ est hydrogène.

13. Composé pour une utilisation selon la revendication 11,
dans lequel R² est un groupe acylamino en C_{2 à 8} comportant un à trois atomes d'halogène.

14. Composé pour une utilisation selon la revendication 11,
dans lequel R³ est hydrogène.

15. Composé pour une utilisation selon la revendication 11,
dans lequel chacun de R⁴ et R⁵ est hydrogène.
